# EUROPEAN PATENT SPECIFICATION

(11) **EP 3 736 276 B1**
(45) Date of publication and mention of the grant of the patent: **01.03.2023**
(21) Application number: 18880604.6
(22) Date of filing: 23.11.2018
(51) Int. Cl.: C07D 333/68, C07D 333/78, C07D 333/80, C07D 495/04, A61K 31/381, A61K 31/382, A61P 29/00, A61P 19/00, A61P 35/00, A61P 35/02, A61P 37/00, A61P 37/02, A61P 37/08

(54) **THIENOCYCLIC COMPOUND AND SYNTHESIS METHOD THEREFOR AND APPLICATION THEREOF**
THIENOCYCLISCHE VERBINDUNG UND SYNTHESEVERFAHREN DAFÜR UND ANWENDUNG DAVON
COMPOSÉ THIÉNOCYCLIQUE, PROCÉDÉ DE SYNTHÈSE CORRESPONDANT ET UTILISATIONS ASSOCIÉES

(30) Priority: 27.11.2017 CN 201711206672
(43) Date of publication of application: 11.11.2020
(73) Proprietor: Shanghai Yuyao Biotech Ltd., Shanghai 201100 (CN)
(72) Inventor: ZHANG, Hankun, Shanghai 200241 (CN); LU, Weiqiang, Shanghai 200214 (CN); LIU, Mingyao, Shanghai 200214 (CN); YANG, Junjie, Shanghai 200214 (CN); YU, Weiwei, Shanghai 200214 (CN); HU, Longlong, Shanghai 200214 (CN); ZHANG, Xinpei, Shanghai 200214 (CN); LIN, Xianhua, Shanghai 200214 (CN); LIU, Wenjuan, Shanghai 200214 (CN); XI, Zaixi, Shanghai 200214 (CN)
(74) Representative: Lavoix
(86) International application number: PCT/CN2018/117235
(87) International publication number: WO 2019/101171

(56) References cited:
- EP-A1- 2 277 858
- WO-A1-2010/019796
- CN-A- 102 026 961
- CN-A- 102 149 384
- DATABASE REGISTRY 24 March 2014 (2014-03-24), retrieved from STN Database accession no. 1572557-47-1
- DATABASE REGISTRY 14 March 2014 (2014-03-14), retrieved from STN Database accession no. 1568977-72-9

## Description

### TECHNICAL FIELD

The present invention belongs to the field of biomedicine, and relates to a class of thienocyclic compounds, their synthesis methods, and use thereof. These thienocyclic compounds reported in the invention are used as prostaglandin receptor EP4 antagonists for preventing and treating prostaglandin PGE2-mediated diseases, including malignant tumors, autoimmune diseases, inflammation, pain, etc.

### BACKGROUND

There is a long history for studying the function of prostaglandin E2 in the regulation of acute and chronic inflammation, and its mechanisms of action was well elaborated. In recent years, growing numbers of regulatory mechanisms of prostaglandin E2 on inflammatory cells and cytokines involved in tumor immunity were revealed.

Among all prostaglandin subtypes, prostaglandin E2 (PGE2) is the most abundant and widely distributed one within the human body and participates in regulating numerous physiological and pathophysiological processes, including inflammation, pain, renal function, cardiovascular system, lung function, and cancer. PGE2 binds to four different subtypes EP1, EP2, EP3 and EP4 (also known as PTGER1, PTGER2, PTGER2, and PTGER4) of G-protein-coupled receptors on the cell surface via autocrine or paracrine manner, and these subtypes of G-protein-coupled receptors exert their biological effects by coupling different G proteins to activate different downstream signaling pathways. Athought both EP2 and EP4 receptor subtypes mediate downstream signals by coupling with the same Gₛ protein, they have some structural differences due to only 31% homology in amino acid sequence. Therefore, there are some similarities but also many defferences in their main physiological functions.

PGE2 plays a very important role in promoting tumor development and progress. Elevated levels of PGE2 and its related receptors EP2 and EP4 are found in various malignancies including colon cancer, lung cancer, breast cancer, and head and neck cancer, and are often correlated with a poor prognosis. The expression of COX2, a PGE2 synthetase regulated by transcription factors HIF1_{α} and SP1, in tumor tissues was obviously increased. While15-PGDH, a PGE2-degrading enzyme, was highly expressed in normal tissues and lacked in colon, gastric, lung and breast cancer tissues, which further results in elevated levels of PGE2 in the tumor microenvironment.

Recently, in order to avoid the cardiovascular side effects caused by COX2 inhibitors, specific antagonists that selectively target PGE2 signals such as PGE2 receptor EP2 and EP4 were developed, and some of them subsequently entered into preclinical and clinical research. Existing preclinical research data indicated that EP2 and EP4 can prevent or inhibit the growth of various tumors to different extents in animal models of colon cancer, esophageal cancer, lung cancer, breast cancer and the like.

EP227858 discloses compounds and a pharmaceutical composition of the compound and the use thereof in the preparation of the pharmaceutical composition for preventing and/or treating prostaglandin PGE2-mediated inflammatory diseases, various types of pain and other diseases.

WO2010/019796 discloses compounds useful as EP4 selective antagonists, and also discloses that the EP4 receptor antagonist can be used in the treatment of liver cancer, lung cancer, breast cancer and other diseases.

Known in the prior art are also compounds as disclosed DATABASE REGISTRY, RN 1572557-47-1 and DATABASE REGISTRY, RN 1572557-47-1, respectively

In summary, it is believed that the development of specific inhibitors based on the PGE2-mediated signaling pathway and its receptors is still in the early stage of research and exploration. There is still a need to further improve the antagonistic activity of existing drugs, as well as develop antagonists with better selectivity and metabolic stability.

### SUMMARY OF THE INVENTION

The purpose of the present invention is to provide EP4 antagonists with improved activity, higher selectivity to subtypes of EP4, shows no inhibitory activity against normal and tumor cells, but able to play a good role in immunoregulatory via blocking the receptor signaling pathways of prostaglandin 2 prostaglandin receptor -4 (PGE₂-EP4), and improved metabolic stability and good hydrophilia that can basically meeting the requirements of druggability.

In the first aspect of the present invention, provided the following compound of formula (I): 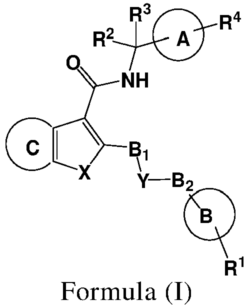 or pharmaceutically acceptable salts or hydrates thereof, wherein:
Ⓐ and Ⓑ are each independently select form the group consisting of C3-C6 carbon ring, benzene ring, 5- or 6-membered heteroaromatic ring having one or more O, N, S atoms, wherein Ⓐ and Ⓑ can be optionally substituted by 1 to 3 R⁵ substitutes;
Ⓒ is a substituted or unsubstituted ring selected from the group consisting of a 4-7 membered saturated heterocyclic ring, , wherein the heterocyclic ring comprises one or more heteroatoms selected from O; the ring can be monocylic, bicyclic, spiro or bridge ring;
X is -S;
Y is absent, or a group selected from the group consisting of -CH₂-, -O-, -S-, -SO-, -SO₂-, and -N(R⁸)-;
B₁ and B₂ are groups each independently selected from the group consisting of absent, C1-C6 alkylene, C2-C6 alkenylene, C2-C6 alkynylene; and B₁, B₂ and Y are not absent at the same time;
R¹ is one or more group selected from the group consisting of H, C1-C6 alkyl, halogen, nitro, -N(R⁹)(R¹⁰), -OH, -CN, C1-C6 haloalkyl (preferably difluoromethyl or trifluoromethyl), C1-C6 alkoxy (preferably methoxy or ethoxy), and C1-C6 haloalkoxy (preferably difluoromethoxy or trifluoromethoxy);
R² and R³ are each independently selected from the group consisting of H, halogen, C1-C6 alkyl, C1-C6 haloalkyl, and C3-C6 cycloalkyl; or R², R³ together with the carbon atom to which they are attached form 3 to 6 membered ring, which is carbon ring or heterocyclic ring including one or more heteroatoms selected from O, S or N(R¹¹);
R⁴ is -COOR¹² (preferably, -COOH, -COOCH₃, -COOCH₂CH₃, -COOCH₂CH₂CH₃, -COOCH(CH₃)₂);
R⁵ is selected from: H, halogen, C1-C6 alkyl, C1-C6 haloalkyl, C3-C6 cycloalkyl, and C1-C6 alkoxy (preferably methoxy, ethoxy);
R¹² is selected from the group consisting of H and C1-C6 alkyl;
R⁸, R⁹, R¹⁰ and R¹¹ are each independently select form H, C1-C6 alkyl, C1-C6 haloalkyl, C3-C6 cycloalkyl, C3-C6 halocycloalkyl, C6-C10 aryl, 5- or 6- membered heteroaryl, 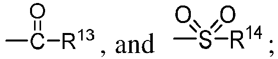
unless otherwise specified, one or more hydrogen atoms of the substituted groups are substituted with substituent(s) selected from the group consisting of: F, Cl, Br, I, hydroxyl, methyl, ethyl, isopropyl, methoxy, ethoxy, trifluoromethyl, difluoromethoxy, trifluoromethoxy, nitro, -CN, oxo (= O);
R¹³ and R¹⁴ are each independently selected from: H, C1-C6 alkyl (preferably methyl, ethyl, propyl, butyl, isopropyl, tert-butyl, pentyl, hexyl), C6-C10 aryl, C1-C6 alkylene, and -C6-C10 aryl.

Preferably, in Formula (I) 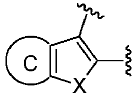 has a structure as shown in following formula: 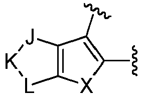
wherein one of J, K, and L is -O-; and the rest of J, K, and L are selected from the group consisting of -CH₂-, -CH(CH₃)-, -CH(CH₂CH₃)-, -C(CH₃)₂-, 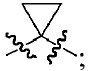 or 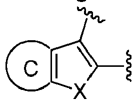 has a structure as shown in following formula: 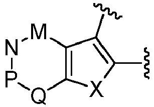
wherein one of M, N, P, and Q is -O-; and the rest of M, N, P, and Q are each independently selected from the group consisting of -CH₂-, -CH(CH₃)-, -CH(CH₂CH₃)-, -C(CH₃)₂-, and 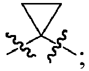 or 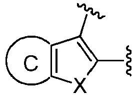 has a structure as shown in following formula: 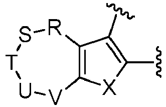
wherein one of R, S, T, U, and V is -O-; and the rest of R, S, T, U, and V are each independently selected from the group consisting of -CH₂-, -CH(CH₃)-, -CH(CH₂CH₃)-, -C(CH₃)₂-, and

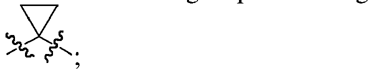

Preferably, M, N, P and Q form a ring structure, or a bridge ring structure consisting of 0-4 carbon atoms is formed between M and Q, M and P, or N and Q;

Preferably, R, S, T, U and V form a ring structure, or a bridge ring structure consisting of 0-4 carbon atoms is formed between any two of R, S, T, U and V.

More preferably, Ⓐ and Ⓑ are each independently selected form the group consisting of benzene ring, and 5- or 6-membered heteroaromatic ring including one or more O, N, S atoms, wherein Ⓐ and Ⓑ can optionally be substituted by 1 to 3 R⁵ substitutes.

More preferably, Ⓒ is a substituted or unsubstituted 6-membered oxa-heterocycle. Preferably, substituted in Ⓒ means one or more (preferably 1 or 2) hydrogen atoms on the ring are substituted by substituent(s) selected from the group consisting of F, Cl, Br, I, hydroxyl, methyl, ethyl, isopropyl, methoxy, ethoxy, trifluoromethyl, difluoromethoxy, trifluoromethoxy, nitro, -CN, oxo (= O); More preferably, substituted by substituent(s) selected from the group consisting of: methyl, ethyl, isopropyl.

Preferably, Y is absent.

Preferably, B₁ and B₂ are each independently selected from the group consisting of -(CH₂)ₙ-; wherein n=0, 1, 2, 3 or 4, -CH=CH-, -CH=CH-CH₂-, -CH₂-CH=CH-, -CH=CH-CH₂-CH₂-, -CH₂-CH=CH-CH₂-, -CH₂-CH₂-CH=CH-; -C≡C-, -C≡C-CH₂-, -CH₂-C≡C-, -C≡C-CH₂-CH₂-, -CH₂-C≡C-CH₂-, -CH₂- CH₂-C≡C-; and B₁ and B₂ are not -(CH₂)ₙ- at the same time, wherein n=0.

More preferably, B1(or B2) is -(CH₂)ₙ-, wherein n=0, and B2 (or B1) is selected from the group consisting of -(CH₂)ₙ-; wherein n=1, 2, 3 or 4, -CH=CH-, -CH=CH-CH₂-, -CH₂-CH=CH-, -CH=CH-CH₂-CH₂-, -CH₂-CH=CH-CH₂-, -CH₂-CH₂-CH=CH-; -C=C-, -C≡C-CH₂-, -CH₂-C≡C-, -C≡C-CH₂-CH₂-, -CH₂-C≡C-CH₂-, -CH₂- CH₂-C≡C-.

Preferably, the compounds are selected from the group consisting of:
(S)-4-(1-(2-((4-fluorophenyl)ethynyl)-5,7-dihydro-4H-thieno[2,3-c]pyran-3-carb oxamido)ethyl)benzoic acid
(S)-4-(1-(2-((4-fluorophenyl)ethynyl)-5,5-dimethyl-5,7-dihydro-4H-thieno[2,3-c] pyran-3-carboxamido)ethyl)benzoic acid
(S)-4-(1-(2-(4-fluorophenethyl)-5,7-dihydro-4H-thieno[2,3-c]pyran-3-carboxami do)ethyl)benzoic acid
(S)-4-(1-(2-(4-fluorophenethyl)-5,5-dimethyl-5,7-dihydro-4H-thieno[2,3-c]pyran-3-carboxamido)ethyl)benzoic acid
(S)-4-(1-(2-(4-(trifluoromethyl)phenethyl)-5,7-dihydro-4H-thieno[2,3-c]pyran-3-carboxamido)ethyl)benzoic acid
(S)-4-(1-(2-(3-(trifluoromethyl)phenethyl)-5,7-dihydro-4H-thieno[2,3-c]pyran-3-carboxamido)ethyl)benzoic acid
(S)-4-(1-(2-(3-fluorophenethyl)-5,7-dihydro-4H-thieno[2,3-c]pyran-3-carboxami do)ethyl)benzoic acid
(S)-4-(1-(2-(4-fluorobenzyl)-5,7-dihydro-4H-thieno[2,3-c]pyran-3-carboxamido) ethyl)benzoic acid
(S)-4-(1-(2-(4-(trifluoromethyl)benzyl)-5,7-dihydro-4H-thieno[2,3-c]pyran-3-car boxamido)ethyl)benzoic acid
(S)-4-(1-(2-(3-(trifluoromethyl)benzyl)-5,7-dihydro-4H-thieno[2,3-c]pyran-3-car boxamido)ethyl)benzoic acid
(R)-4-(1-(2-(4-fluorobenzyl)-5,7-dihydro-4H-thieno[2,3-c]pyran-3-carboxamido) ethyl)benzoic acid
(R)-4-(1-(2-(4-(trifluoromethyl)benzyl)-5,7-dihydro-4H-thieno[2,3-c]pyran-3-car boxamido)ethyl)benzoic acid
(R)-4-(1-(2-(3-(trifluoromethyl)benzyl)-5,7-dihydro-4H-thieno[2,3-c]pyran-3-car boxamido)ethyl)benzoic acid
4-((2-(4-fluorobenzyl)-4,7-dihydro-5H-thieno[2,3-c]pyran-3-carboxamido)methyl )benzoic acid
(S)-4-(1-(2-((4-(trifluoromethyl)benzyl) amino)-5,7-dihydro-4H-thieno[2,3-c]pyran-3-carboxamido)ethyl)benzoic acid
(S)-4-(1-(2-(4-methoxyphenethyl)-4,7-dihydro-5H-thieno[2,3-c]pyran-3-carboxa mido)ethyl)benzoic acid
(S)-4-(1-(2-(3-fluorobenzyl)-4,7-dihydro-5H-thieno[2,3-c]pyran-3-carboxamido) ethyl)benzoic acid
(S)-4-(1-(2-(4-chlorobenzyl)-4,7-dihydro-5H-thieno[2,3-c]pyran-3-carboxamido) ethyl)benzoic acid
(S)-4-(1-(2-(3-fluoro-4-methoxybenzyl)-4,7-dihydro-5H-thieno[2,3-c]pyran-3-car boxamido)ethyl)benzoic acid
(S)-4-(1-(2-(3-chlorobenzyl)-4,7-dihydro-5H-thieno[2,3-c]pyran-3-carboxamido) ethyl)benzoic acid
(S)-4-(1-(2-(3,4-difluorobenzyl)-4,7-dihydro-5H-thieno[2,3-c]pyran-3-carboxami do)ethyl)benzoic acid
(S)-4-(1-(2-(4-methoxybenzyl)-4,7-dihydro-5H-thieno[2,3-c]pyran-3-carboxamid o)ethyl)benzoic acid
((2-(3-(trifluoromethyl)benzyl)-4,7-dihydro-5H-thieno[2,3-c]pyran-3-carboxamid o)methyl)benzoic acid
4-((2-(4-fluorobenzyl)-4,7-dihydro-5H-thieno[2,3-c]pyran-3-carboxamido)methyl )cyclohexane-1-carboxylic acid (racemates)
(1-(2-(4-fluorobenzyl)-4,7-dihydro-5H-thieno[2,3-c]pyran-3-carboxamido)cyclop ropyl)benzoic acid

Preferably, the compounds are selected from the group consisted of YJ101, YJ105, YJ106, YJ110, YJ111, YJ112, YJ113, YJ114, YJ115, YJ116, YJ117, YJ118, YJ119, YJ120, YJ121, YJ122, YJ123, YJ124, YJ125, YJ126, YJ127, YJ128, YJ129, YJ130, YJ131.

Also preferably, the pharmaceutically acceptable salt refers to acid addition salt(s) formed by thienocyclic compound(s) and acid(s), or base addition salt(s) formed by thienocyclic compound(s) and base(s); wherein, the acids include hydrochloric acid, hydrobromic acid, sulfuric acid, phosphoric acid, acetic acid, tartaric acid, salicylic acid, citric acid, malic acid, methanesulfonic acid, *p*-toluenesulfonic acid, lactic acid, pyruvic acid, maleic acid, succinic acid, salts derived from inorganic bases include aluminum, ammonium, potassium, sodium, ferric, copper, calcium, ferrous, magnesium, lithium, manganese, salts derived from organic non-toxic bases include primary, secondary, and tertiary amine salts, substituted amines include natural substituted amines, cyclic amines, ethanolamines, N-ethylmorpholine, N-ethylpiperidine, glucosamine.

The another aspect of the present invention provides a pharmaceutical composition which comprises: a therapeutically effective amount of the compound of formula (I), or pharmaceutically acceptable salts or hydrates thereof; and pharmaceutically acceptable carriers.

In another preferred embodiment, the pharmaceutical composition is in the dosage form selected from the group consisting of injectable fluids, aerosols, creams, gels, pills, capsules, syrups, transdermal patches, or excipients.

The another aspect of the present invention provides the compound of Formula (I), or pharmaceutically acceptable salts or hydrates thereof for use for:
(i) antagonizing prostaglandin receptor -4 (EP4);
(ii) inhibiting prostaglandin PGE 2;
(iii) enhancing *in vivo* immunocompetence of human or mammals, or preventing and /or treating prostaglandin PGE2-mediated diseases;
(iv) reducing side effects of non-steroidal anti-inflammatory drugs and cyclooxygenase-2 inhibitors against cardiovascular systems and gastrointestinal systems.

Preferably, the prostaglandin PGE2-mediated diseases are selected from the group consisting of autoimmune diseases, allergy, inflammation, bone diseases, acute or chronic pain, and tumor.

More preferably, the tumor is selected from the group consisting of: liver cancer, lung cancer, prostate cancer, skin cancer, colon cancer, pancreatic cancer, breast cancer, leukemia, lymphoma, ovarian cancer, stomach cancer, bladder cancer, kidney cancer, oral cancer, melanoma, esophageal cancer, lymphoma, and cervical cancer;

More preferably, the pharmaceutical composition is used for inhibiting the proliferation, growth, infiltration and migration of tumor cells.

### DESCRIPTION OF THE DRAWINGS

FIG. 1 shows concentration-response curves of certain compounds of the present invention against cAMP upregulation induced by human prostaglandin E2 receptor EP4 subtype, wherein A is E7046, B is YJ114.
FIG. 2 shows the evaluation of the effect of compound YJ114 of present invention on the differentiation of mouse bone myeloid cells.
FIG.3 is a graph showing the inhibitory effect of compounds YJ106, YJ114, YJ115, YJ116 and YJ120 of the present invention on tumor growth.
FIG. 4 is a graph showing the inhibitory effect of compound YJ114 of the present invention on the tumor growth of mouse tumor MC38, Pan02, 4T1 and RM-1 cells in subcutaneous tumor-bearing model.

### DETAILED DESCRIPTION

On the basis of long-term and intensive research, the inventors unexpectedly discovered a series of thienocyclic compounds. These compounds can be used as prostaglandin EP4 receptor antagonists for the prevention and treatment of prostaglandin E2 signaling-related diseases. In addition, the compounds reported in the invention have high selectivity. The compounds of the invention have high inhibitory activity against the EP4 receptors and no antagonistic activity against EP1, EP2, and EP3 receptors. Based on the above discoveries, the inventors completed the invention.

### Thienocyclic compounds and pharmaceutically acceptable salts or hydrates thereof

In a preferred embodiment, provided herein is a compound of Formula (I): 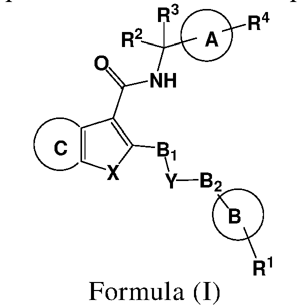 or pharmaceutically acceptable salts or hydrates thereof, wherein:
Ⓐand Ⓑ are each independently select form the group consisting of C3-C6 carbon ring, benzene ring, 5- or 6-membered heteroaromatic ring having one or more O, N, S atoms, wherein Ⓐ and Ⓑ can be optionally substituted by 1 to 3 R⁵ substituents;
Ⓒ is a substituted or unsubstituted ring selected from a 4-7 membered saturated heterocyclic ring, wherein the heterocyclic ring comprises one or more heteroatoms selected from O; the ring can be monocylic, bicyclic, spiro or bridge ring;
X is -S-
Y is absent, or a group selected from the group consisting of -CH₂-, -O-, -S-, -SO-, -SO₂-, and -N(R⁸)-;
B₁ and B₂ are groups each independently selected from the group consisting of absent, C1-C6 alkylene, C2-C6 alkenylene, C2-C6 alkynylene; preferably, B₁ and B₂ are each independently selected from the group consisting of -(CH₂)ₙ-, wherein n = 0, 1, 2, 3 or 4, -CH=CH-, -CH=CH-CH₂-, -CH₂-CH=CH-, -CH=CH-CH₂-CH₂-, -CH₂-CH=CH-CH₂-, -CH₂-CH₂-CH=CH-; -C=C-, -C≡C-CH₂-, -CH₂-C≡C-, -C≡C-CH₂-CH₂-, -CH₂-C≡C-CH₂-, -CH₂-CH₂-C≡C-; and B₁, B₂ and Y are not absent at the same time;
R¹ is one or more group selected from the group consisting of H, C1-C6 alkyl, halogen, nitro, -N(R⁹)(R¹⁰), -OH, -CN, C1-C6 haloalkyl (preferably difluoromethyl or trifluoromethyl), C1-C6 alkoxy (preferably methoxy or ethoxy), and C1-C6 haloalkoxy (preferably difluoromethoxy or trifluoromethoxy);
R² and R³ are each independently selected from the group consisting of H, halogen, C1-C6 alkyl, C1-C6 haloalkyl, and C3-C6 cycloalkyl; or R², R³ together with the carbon atom to which they are attached form 3 to 6 membered ring, which is carbon ring or heterocyclic ring including one or more heteroatoms selected from O, S or N(R¹¹);
R⁴ is -COOR¹² (preferably, -COOH, -COOCH₃, -COOCH₂CH₃, -COOCH₂CH₂CH₃, -COOCH(CH₃)₂);
R⁵ is selected from: H, halogen, C1-C6 alkyl, C1-C6 haloalkyl, C3-C6 cycloalkyl, and C1-C6 alkoxy (preferably methoxy, ethoxy);
R¹² is selected from the group consisting of H and C1-C6 alkyl;
R⁸, R⁹, R¹⁰ and R¹¹ are each independently select form H, C1-C6 alkyl, C1-C6 haloalkyl, C3-C6 cycloalkyl, C3-C6 halocycloalkyl, C6-C10 aryl, 5- or 6- membered heteroaryl, 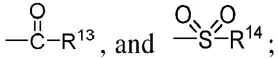
unless otherwise specified, one or more hydrogen atoms of the substituted groups are substituted with substituent(s) selected from the group consisting of: F, Cl, Br, I, hydroxyl, methyl, ethyl, isopropyl, methoxy, ethoxy, trifluoromethyl, difluoromethoxy, trifluoromethoxy, nitro, -CN, oxo (= O);
R¹³ and R¹⁴ are each independently selected from: H, C1-C6 alkyl (preferably methyl, ethyl, propyl, butyl, isopropyl, tert-butyl, pentyl, hexyl), C6-C10 aryl, C1-C6 alkylene, and -C6-C10 aryl.

In another preferred embodiment, in formula (I) 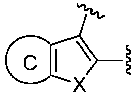 has a structure as shown in following Formula: 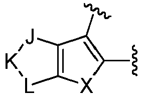
wherein one of J, K, and L is -O-; and the rest of J, K, and L are selected from the group consisting of -CH₂-, -CH(CH₃)-, -CH(CH₂CH₃)-, -C(CH₃)₂-, 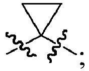 or 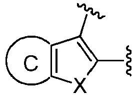 has a structure as shown in following formula: 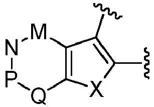
wherein one of M, N, P, and Q is -O-; and the rest of M, N, P, and Q are each independently selected from the group consisting of -CH₂-, -CH(CH₃)-, -CH(CH₂CH₃)-, -C(CH₃)₂-, and 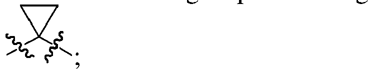
or 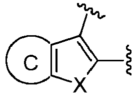 has a structure as shown in following formula: 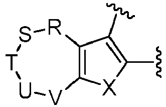
wherein one of R, S, T, U, and V is -O-; and the rest of R, S, T, U, and V are each independently selected from the group consisting of -CH₂-, -CH(CH₃)-, -CH(CH₂CH₃)-, -C(CH₃)₂-, and 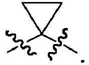
In a preferred embodiment, M, N, P and Q form a ring structure, or a bridge ring structure consisting of 0-4 carbon atoms is formed between M and Q, M and P, or N and Q;
In a preferred embodiment, R, S, T, U and V form a ring structure, or a bridge ring structure consisting of 0-4 carbon atoms is formed between any two of R, S, T, U and V.

In a preferred embodiment, Ⓐ and Ⓑ are each independently selected form the group consisting of: benzene ring, 5- or 6-membered heteroaromatic ring including one or more O, N, S atoms, wherein Ⓐ and Ⓑ can be optionally substituted with 1 to 3 R⁵ substitutes.

In a preferred embodiment, the compounds are selected from the group consisted of YJ101, YJ105, YJ106, YJ110, YJ111, YJ112, YJ113, YJ114, YJ115, YJ116, YJ117, YJ118, YJ119, YJ120, YJ121, YJ122, YJ123, YJ124, YJ125, YJ126, YJ127, YJ128, YJ129, YJ130, YJ131.

In another preferred embodiment, the pharmaceutically acceptable salt refers to acid addition salt(s) formed by thienocyclic compound(s) and acid(s), or base addition salt(s) formed by thienocyclic compound(s) and base(s); wherein, the acids include hydrochloric acid, hydrobromic acid, sulfuric acid, phosphoric acid, acetic acid, tartaric acid, salicylic acid, citric acid, malic acid, methanesulfonic acid, p-toluenesulfonic acid, lactic acid, pyruvic acid, maleic acid, succinic acid, salts derived from inorganic bases include aluminum, ammonium, potassium, sodium, ferric, copper, calcium, ferrous, magnesium, lithium, manganese, salts derived from organic non-toxic bases include primary, secondary, and tertiary amine salts, substituted amines include natural substituted amines, cyclic amines, ethanolamines, N-ethylmorpholine, N-ethylpiperidine, glucosamine.

### Pharmaceutical composition

In a preferred embodiment, the pharmaceutical composition comprises: a therapeutically effective amount of the compound of formula (I), or pharmaceutically acceptable salts or hydrates thereof; and pharmaceutically acceptable carriers.

In another preferred embodiment, the pharmaceutical composition is in the dosage form selected from the group consisted of injectable fluids, aerosols, creams, gels, pills, capsules, syrups, transdermal patches, or excipients.

### The use of thienocyclic compounds

In a preferred embodiment, provided herein is the compound of Formula (I), or pharmaceutically acceptable salts or hydrates thereof for use for:
(i) antagonizing prostaglandin receptor -4 (EP4);
(ii) inhibiting prostaglandin PGE 2;
(iii) enhancing immunocompetence *in vivo* of human or mammals, or preventing and /or treating prostaglandin PGE2-mediated diseases;
(iv) reducing side effects of non-steroidal anti-inflammatory drugs and cyclooxygenase-2 inhibitors against cardiovascular systems and gastrointestinal systems.

In a preferred embodiment, in the use, the prostaglandin PGE2-mediated diseases are selected from the group consisting of autoimmune diseases, allergy, inflammation, bone diseases, acute or chronic pain, and tumor.

More preferably, the tumor is selected from the group consisting of: liver cancer, lung cancer, prostate cancer, skin cancer, colon cancer, pancreatic cancer, breast cancer, leukemia, lymphoma, ovarian cancer, stomach cancer, bladder cancer, kidney cancer, oral cancer, melanoma, esophageal cancer, lymphoma, and cervical cancer;

In a preferred embodiment, the pharmaceutical composition is used for inhibiting the proliferation, growth, infiltration and migration of tumor cells.

### TERMS

In the present invention, term "C3-C6 carbon ring" or term "C4-C7 carbon ring" means saturated or unsaturated rings consisting of 3 to 6 carbon atoms or 4 to 7 carbon atoms, including monocylic, bicyclic, spiro or bridge rings, e.g. 6-membered aliphatic ring.

The term "5- or 6-membered heteroaromatic ring" refers to a 5- to 6-membered aromatic ring having one or more heteroatoms selected from nitrogen, oxygen, or sulfur, for example, pyridine, pyrimidine, thiazole, isothiazole, furan, thiophene, pyrrole.

The term "C1-C6 alkyl" refers to a linear or branched alkyl group having 1 to 6 carbon atoms, including, without limitation, methyl, ethyl, propyl, isopropyl, butyl, isobutyl, Sec-butyl, tert-butyl, pentyl and hexyl and the like; preferably ethyl, propyl, isopropyl, butyl, isobutyl, sec-butyl and tert-butyl.

The term "C1-C6 alkylene" refers to a linear or branched alkylene group having 1 to 6 carbon atoms, including, without limitation, methylene, ethylene, propylene, isopropylene, butylene, isobutylene, sec-butylene, tert-butylene, pentylene, hexylene and the like; preferably ethylene, propylene, isopropylene, butylene, isobutylene, sec-butylene and tert-butylene.

The term "C2-C6 alkenylene"refers to a linear or branched alkenylene group having 2 to 6 carbon atoms and containing a double bond, including without limitation ethenylidene, propenylidene, butenylidene, isobutenylidene, pentenylidene and hexynylene and the like.

The term "C2-C6 alkynylene"refers to a linear or branched alkynylene group having 2 to 6 carbon atoms and containing a double bond, including without limitation ethynylene, propinylene, butynlene, isobutynlene, entynylene and hexynylene and the like.

The term "C₃-C₆ cycloalkyl" refers to a cyclic alkyl group having 3 to 10 carbon atoms on the ring including, without limitation, cyclopropyl, cyclobutyl, cyclopentyl, and cyclohexyl.

The term "C6-C10 aryl" refers to an aromatic ring group having 6 to 10 carbon atoms and without any heteroatoms on the ring, such as phenyl.

The terms "C1-C6 haloalkyl", "C1-6 halocycloalkyl" refer to the group in which the hydrogens on the alkyl or cycloalkyl are substituted with one or more halogen atoms, including, without limitation, -CHF₂, chlorocyclopropyl and the like.

The term "C1-C6 alkoxy" refers to a linear or branched alkoxy group having 1 to 6 carbon atoms, including, without limitation, methoxy, ethoxy, propoxy, isopropoxy and butoxy.

The term "C1-C6 haloalkoxy" refers to the group in which the hydrogens on the alkoxy group are substituted with one or more halogen atoms.

### Compound of Formula I

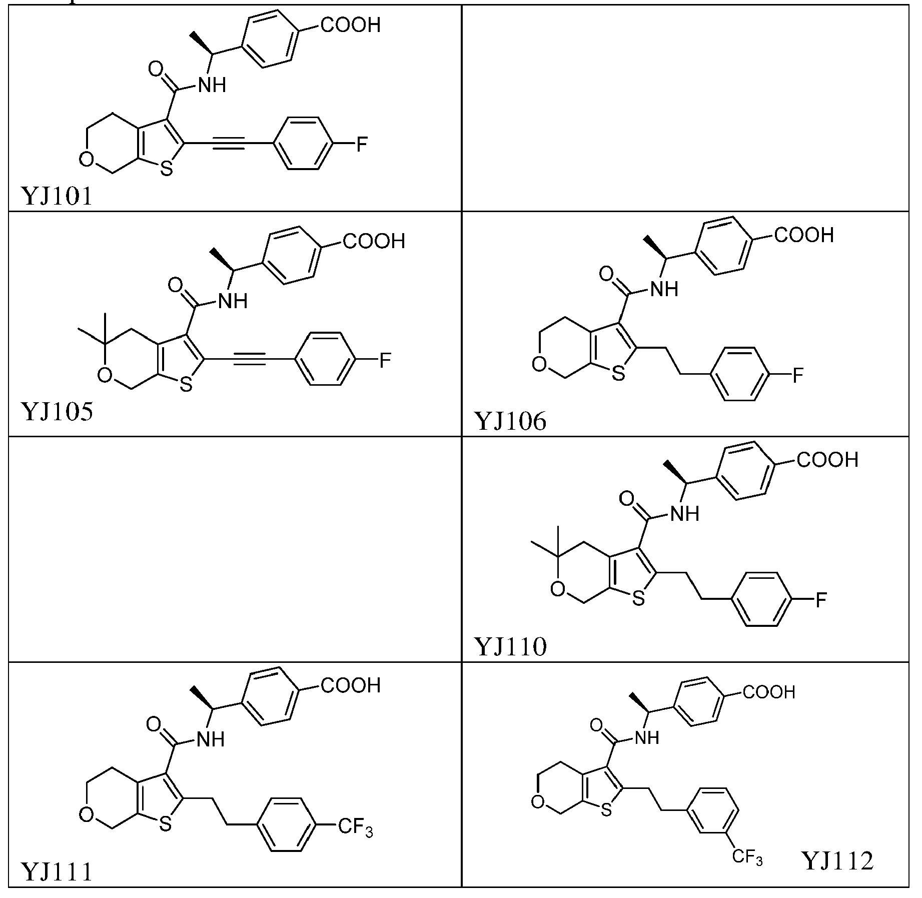

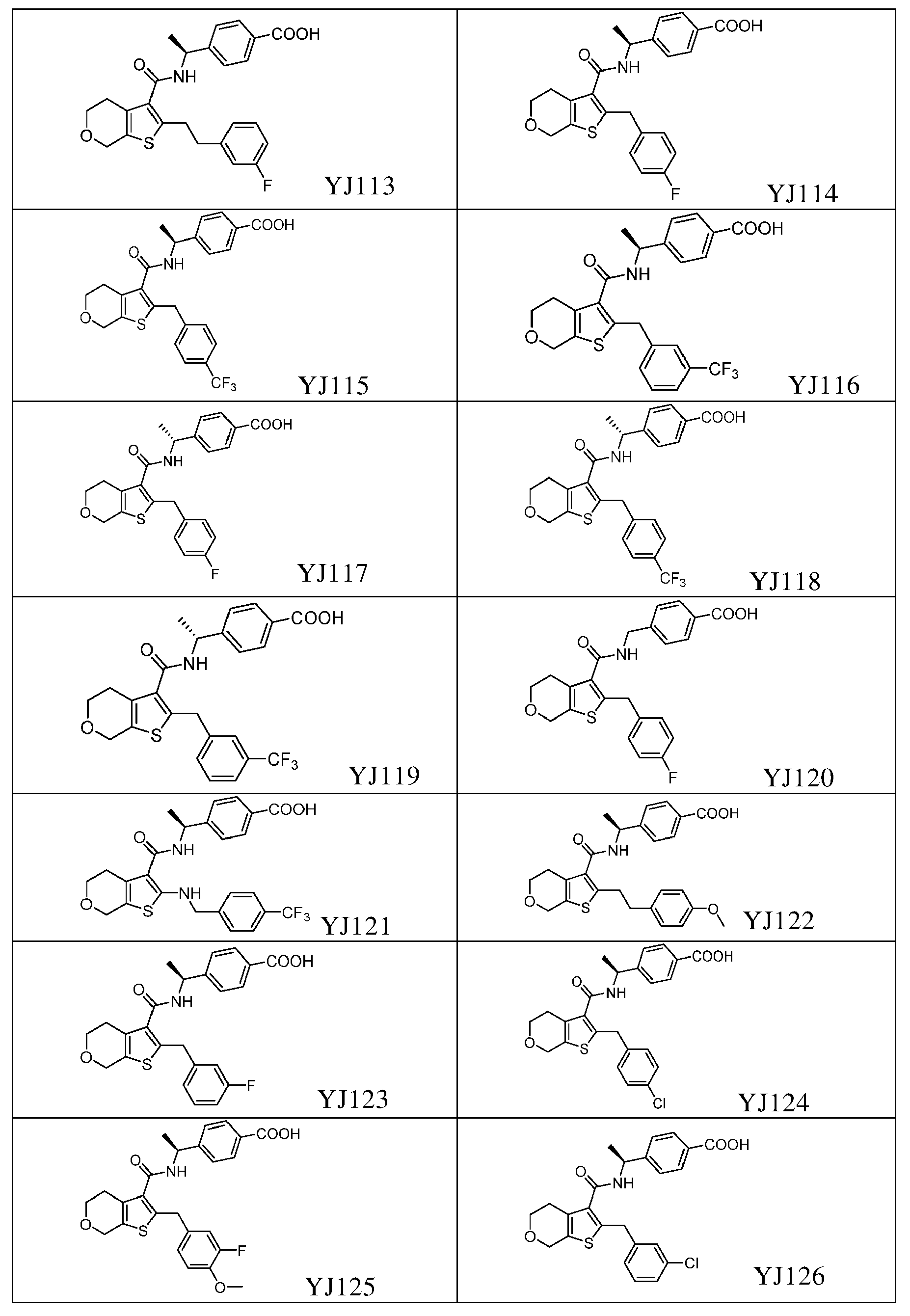

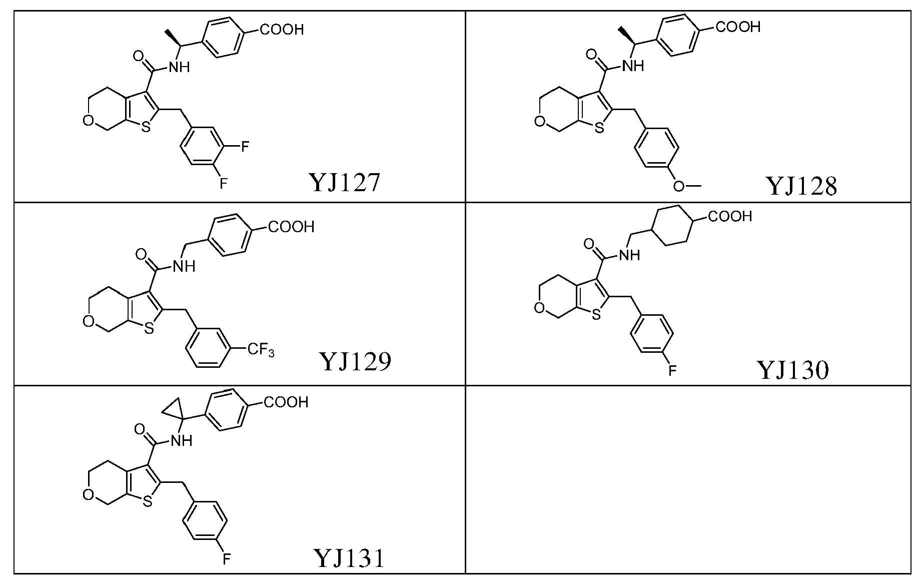

### Preparation of Compound of Formula I

Preparing processes for exemplary compounds of formula I are shown in the following Table 1:

**Table 1**

| | Name | Preparing Process |
|---|---|---|
| YJ101 | (S)-4-(1-(2-((4-fluorophenyl)ethyny 1)-5,7-dihydro-4H-thieno[2,3-c]pyra n-3-carboxamido)ethyl)benzoic acid | As shown in Example 1-1 |
| YJ102 | (S)-4-(1-(2-((4-fluorophenyl)ethyny 1)-5,6-dihydro-4H-cyclopenta[b]thio phene-3-carboxamido)ethyl)benzoic acid (not part of the invention) | Prepared according to the preparation of Compound YJ101 wherein tetrahydro-4H-pyran-4-one is replaced with cyclopentanone |
| YJ103 | 4-((1S)-1-(6-ethyl-2-((4-fluorophen yl)ethynyl)-4,5,6,7-tetrahydrobenzo[ b]thiophene-3-carboxamido)ethyl)b enzoic acid (not part of the invention) | Prepared according to the preparation of Compound YJ101 wherein tetrahydro-4H-pyran-4-one is replaced with 4-ethylcyclohexanone |
| YJ104 | (S)-4-(1-(2-((4-fluorophenyl)ethyny 1)-5,6,7,8-tetrahydro-4H-cyclohepta[ b]thiophene-3-carboxamido)ethyl)b enzoic acid (not part of the invention) | Prepared according to the preparation of Compound YJ101 wherein tetrahydro-4H-pyran-4-one is replaced with cycloheptanone |
| YJ105 | (S)-4-(1-(2-((4-fluorophenyl)ethyny 1)-5,5-dimethyl-5,7-dihydro-4H-thie no[2,3-c]pyran-3-carboxamido)ethyl )benzoic acid | Prepared according to the preparation of Compound YJ101 wherein tetrahydro-4H-pyran-4-one is replaced with tetrahydro-2,2-dimethyl-4H-pyra n-4-one |
| YJ106 | (S)-4-(1-(2-(4-fluorophenethyl)-5,7-dihydro-4H-thieno [2,3-c]pyran-3-ca rboxamido)ethyl)benzoic acid | As shown in Example 1-6 |
| YJ107 | (S)-4-(1-(2-(4-fluorophenethyl)-5,6, 7,8-tetrahydro-4H-cyclohepta[b]thio phene-3-carboxamido)ethyl)benzoic acid (not part of the invention) | Prepared according to the method for Compound YJ106 wherein tetrahydro-4H-pyran-4-one is replaced with cycloheptanone |
| YJ108 | (S)-4-(1-(2-(4-fluorophenethyl)-4,5, 6,7-tetrahydrobenzo[b]thiophene-3-carboxamido)ethyl)benzoic acid (not part of the invention) | Prepared according to the method for Compound YJ106 wherein tetrahydro-4H-pyran-4-one is replaced with cyclohexanone |
| YJ109 | (S)-4-(1-(2-(4-fluorophenethyl)-5,6-dihydro-4H-cyclopenta[b]thiophene -3-carboxamido)ethyl)benzoic acid (not part of the invention) | Prepared according to the method for Compound YJ106 wherein tetrahydro-4H-pyran-4-one is replaced with cyclopentanone |
| YJ110 | (S)-4-(1-(2-(4-fluorophenethyl)-5,5-dimethyl-5,7-dihydro-4H-thieno[2,3 -c]pyran-3-carboxamido)ethyl)benz oic acid | Prepared according to the method for Compound YJ106 wherein tetrahydro-4H-pyran-4-one is replaced with tetrahydro-2,2-dimethyl-4H-pyra n-4-one |
| YJ111 | (S)-4-(1-(2-(4-(trifluoromethyl)phen ethyl)-5,7-dihydro-4H-thieno[2,3-c] pyran-3-carboxamido)ethyl)benzoic acid | Prepared according to the method for Compound YJ106 wherein 1-ethynyl-4-fluorobenzene is replaced with 1-ethynyl-4-(trifluoromethyl)benzene |
| YJ112 | (S)-4-(1-(2-(3-(trifluoromethyl)phen ethyl)-5,7-dihydro-4H-thieno[2,3-c] pyran-3-carboxamido)ethyl)benzoic acid | Prepared according to the method for Compound YJ106 wherein 1-ethynyl-4-fluorobenzene is replaced with 1-ethynyl-3-(trifluoromethyl)benzene |
| YJ113 | (S)-4-(1-(2-(3-fluorophenethyl)-5,7-dihydro-4H-thieno [2,3-c]pyran-3-ca rboxamido)ethyl)benzoic acid | Prepared according to the method for Compound YJ106 wherein 1-ethynyl-4-fluorobenzene is replaced with 1-ethynyl-3-fluorobenzene |
| YJ114 | (S)-4-(1-(2-(4-fluorobenzyl)-5,7-dih ydro-4H-thieno [2,3-c]pyran-3-carbo xamido)ethyl)benzoic acid | As shown in Example 1-14 |
| YJ115 | (S)-4-(1-(2-(4-(trifluoromethyl)benz yl)-5,7-dihydro-4H-thieno[2,3-c]pyr an-3-carboxamido)ethyl)benzoic acid | Prepared according to the method for Compound YJ114 wherein p-fluorobenzaldehyde is replaced with p-trifluoromethyl benzaldehyde |
| YJ116 | (S)-4-(1-(2-(3-(trifluoromethyl)benz yl)-5,7-dihydro-4H-thieno[2,3-c]pyr an-3-carboxamido)ethyl)benzoic acid | Prepared according to the method for Compound YJ114 wherein -fluorobenzaldehyde is replaced with 3-trifluoromethyl benzaldehyde |
| YJ117 | (R)-4-(1-(2-(4-fluorobenzyl)-5,7-dih ydro-4H-thieno [2,3-c]pyran-3-carbo xamido)ethyl)benzoic acid | Prepared according to the method for Compound YJ114 wherein methyl (S)-4-(1-aminoethyl)benzoate is replaced with methyl (R)-4-(1-aminoethyl)benzoate |
| YJ118 | (R)-4-(1-(2-(4-(trifluoromethyl)benz yl)-5,7-dihydro-4H-thieno[2,3-c]pyr an-3-carboxamido)ethyl)benzoic acid | Prepared according to the method for Compound YJ114 wherein methyl (S)-4-(1-aminoethyl)benzoate is replaced with methyl (R)-4-(1-aminoethyl)benzoate and p-fluorobenzaldehyde is replaced with p-trifluoromethyl benzaldehyde |
| YJ119 | (R)-4-(1-(2-(3-(trifluoromethyl)benz yl)-5,7-dihydro-4H-thieno[2,3-c]pyr an-3-carboxamido)ethyl)benzoic acid | Prepared according to the method for Compound YJ114 wherein p-fluorobenzaldehyde is replaced with 3-trifluoromethylbenzaldehyde, and in the subsequent steps, methyl (S)-4- (1-aminoethyl)benzoate is replaced with methyl (R)-4-(1-aminoethyl)benzoate |
| YJ120 | 4-((2-(4-fluorobenzyl)-4,7-dihydro-5H-thieno[2,3-c]pyran-3-carboxami do)methyl)benzoic acid | Prepared according to the method for Compound YJ114 wherein methyl (S)-4-(1-aminoethyl)benzoate is replaced with methyl 4-aminomethylbenzoate |
| YJ121 | (S)-4-(1-(2-((4-(trifluoromethyl)ben zyl) amino)-5,7-dihydro-4H-thieno[2,3-c ]pyran-3-carboxamido)ethyl)benzoi c acid | As shown in Example 1-21 |
| YJ122 | (S)-4-(1-(2-(4-methoxyphenethyl)-4 ,7-dihydro-5H-thieno[2,3-c]pyran-3-carboxamido)ethyl)benzoic acid | Prepared according to the method for Compound YJ106 wherein 1-ethynyl-4-fluorobenzene is replaced with 1-ethynyl-3-(methoxyl)benzene |
| YJ123 | (S)-4-(1-(2-(3-fluorobenzyl)-4,7-dih ydro-5H-thieno[2,3-c]pyran-3-carbo xamido)ethyl)benzoic acid | Prepared according to the method for Compound YJ114 wherein p-fluorobenzaldehyde is replaced with m-fluorobenzaldehyde |
| YJ124 | (S)-4-(1-(2-(4-chlorobenzyl)-4,7-dih ydro-5H-thieno[2,3-c]pyran-3-carbo xamido)ethyl)benzoic acid | Prepared according to the method for Compound YJ114 wherein p-fluorobenzaldehyde is replaced with p-chlorobenzaldehyde |
| YJ125 | (S)-4-(1-(2-(3-fluoro-4-methoxyben zyl)-4,7-dihydro-5H-thieno[2,3-c]py ran-3-carboxamido)ethyl)benzoic acid | Prepared according to the method for Compound YJ114 wherein p-fluorobenzaldehyde is replaced with 3-fluoro-4-methoxybenzaldehyde |
| YJ126 | (S)-4-(1-(2-(3-chlorobenzyl)-4,7-dih ydro-5H-thieno[2,3-c]pyran-3-carbo xamido)ethyl)benzoic acid | Prepared according to the method for Compound YJ114 wherein p-fluorobenzaldehyde is replaced with m-chlorobenzaldehyde |
| YJ127 | (S)-4-(1-(2-(3,4-difluorobenzyl)-4,7 -dihydro-5H-thieno[2,3-c]pyran-3-c arboxamido)ethyl)benzoic acid | Prepared according to the method for Compound YJ114 wherein p-fluorobenzaldehyde is replaced with 3,4-difluorobenzaldehyde |
| YJ128 | (S)-4-(1-(2-(4-methoxybenzyl)-4,7-dihydro-5H-thieno[2,3-c]pyran-3-ca rboxamido)ethyl)benzoic acid | Prepared according to the method for Compound YJ114 wherein p-fluorobenzaldehyde is replaced with 4-methoxybenzaldehyde |
| YJ129 | ((2-(3-(trifluoromethyl)benzyl)-4,7-dihydro-5H-thieno[2,3-c]pyran-3-ca rboxamido)methyl)benzoic acid | Prepared according to the method for Compound YJ120 wherein p-fluorobenzaldehyde is replaced with 3-trifluoromethylbenzaldehyde |
| YJ130 | 4-((2-(4-fluorobenzyl)-4,7-dihydro-5H-thieno[2,3-c]pyran-3-carboxami do)methyl)cyclohexane-1-carboxyli c acid (racemates) | Prepared according to the method for Compound YJ114 wherein methyl (S)-4-(1-aminoethyl)benzoate is replaced with methyl 4-(aminomethyl)cyclohexane-1-c arboxylate hydrochloride |
| YJ131 | (1-(2-(4-fluorobenzyl)-4,7-dihydro-5H-thieno[2,3-c]pyran-3-carboxami do)cyclopropyl)benzoic acid | Prepared according to the method for Compound YJ114 wherein methyl (S)-4-(1-aminoethyl)benzoate is replaced with methyl 4-(1-aminocyclopropyl)benzoate |

### Pharmaceutical Compositions and Applications

Another aspect of the present invention provides a pharmaceutical composition containing a therapeutically effective amount of one or more compounds selected from the above formula (I), pharmaceutically acceptable salts, enantiomers, diastereomers or racemates thereof, and optionally, one or more pharmaceutically acceptable carriers, excipients, adjuvants, ingredients, and/or diluents. The ingredients are, for example, odorants, flavors, sweeteners and the like.

The pharmaceutical compositions provided by the invention preferably contain the active ingredient in a weight ratio of 1-99%. Its preferred ratio was that the compounds of the general formula I as active ingredient accounts for 65% to 99% by weight of the total weight, and the remainder are pharmaceutically acceptable carriers, diluents or solutions or saline solutions.

The compounds and pharmaceutical compositions provided by the invention can be in various forms, such as tablets, capsules, powders, syrups, solutions, suspensions, and aerosols, etc, and can be stocked in a suitable solid or liquid carriers or diluents and in suitable disinfection devices for injection or drip infusion.

Various dosage forms of the pharmaceutical compositions of the invention can be prepared according to the conventional preparation methods in the pharmaceutical field. The unit dose of the formulation contains 0.05-200 mg of the compounds of the formula I. Preferably, the unit dose of the formulation contains 0.1 mg - 100 mg of the compounds of the formula I.

The compounds and pharmaceutical compositions of the invention can be used clinically in mammals, including humans and animals and can be administered by mouth, nose, skin, lung, or gastrointestinal tract. Most preferred was oral. Most preferred daily dose is of 0.01-200 mg / kg body weight, one-time administration, or 0.01-100 mg / kg body weight in divided doses. Regardless of any method of administration, the optimal dosages for an individual should be determined depend on the specific treatment. Normally, start with a small dose and gradually increase the dose until the most suitable dose is found.

The invention was further described hereafter in combination with specific embodiments. The experimental methods without specific conditions in the following examples generally follow the conventional conditions or the conditions suggested by the manufacturer. Unless otherwise indicated, percentages and parts are by weight.

In the following preparation examples, ¹H NMR are measured using Bruker 500 MHz instrument and MS are measured usingBruker MicroTOF-Q II LCMS instrument, unless otherwise indicated, measured in ESI mode. All solvents are re-distilled before use and the anhydrous solvents are obtained by drying in accordance with standard methods. Unless otherwise specified, all reactions are conducted under argon atmosphere and tracked with TLC and in post-treatment, being washed with saturated brine and dried over anhydrous magnesium sulfate. Unless otherwise specified, the products were purified by column chromatography on silica gel (200-300 mesh). The silica gel used including 200-300 mesh and GF254 are produced by Qingdao Haiyang Chemical or Yantai Yuanbo Silica Gel Company.

### Example 1-1. Preparation of (S)-4-(1-(2-((4-fluorophenyl)ethynyl)-5,7-dihydro-4H-thieno[2,3-c]pyran-3-carboxam ido)ethyl)benzoic acid (YJ101).

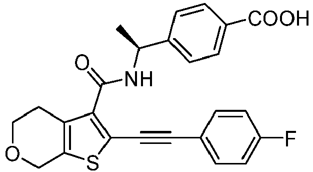

Tetrahydro-4H-pyran-4-one (2.00 g, 20.0 mmol), ethyl cyanoacetate (2.50 g, 22.0 mmol) and sulphur (704 mg, 22.0 mmol) were dissolved in 30.0 mL ethyl alcohol, then the solution was added morpholine (1.74 g, 20.0 mmol) , and stirred at 50 °C overnight. The reaction was monitored by TLC. After the completion of the reaction, the reaction solution was extracted with ethyl acetate and water and the upper organic phase was obtained and evaporated to dryness, purified by column chromatography to afford pale yellow solid, i.e. ethyl
2-amino-5,7-dihydro-4H-thieno[2,3-c]pyran-3-carboxylate (4.38 g, yield 96%). Ethyl 2-amino-5,7-dihydro-4H-thieno[2,3-c]pyran-3-carboxylate (450 mg, 2.0 mmol) was dissolved in 1.5 M HCl (10.0 mL), and stirred for 20 min at room temperature, then cooled to 0 °C in an ice bath. NaNO₂ (207 mg, 3.0 mmol) was added to the solution and the mixture was stirred for 30 min under ice bath. Subsequently, KI (830 mg, 5.0 mmol) was added to the reaction solution in portions, and continued to react at 0 °C for 45 min. After the completion of the reaction, the reaction solution was extracted with water and ethyl acetate, and the organic phase was evaporated to dryness and purified by column chromatography to afford pale yellow solid, i.e. ethyl
2-iodo-5,7-dihydro-4H-thieno[2,3-c]pyran-3-carboxylate as (175 mg, yield 26%). Ethyl 2-iodo-5,7-dihydro-4H-thieno[2,3-c]pyran-3-carboxylate (160 mg, 0.48 mmol), 1-ethynyl-4-fluorobenzene (86 mg, 0.71 mmol), 10% Pd/C (5 mg, 0.048 mmol), PPh₃ (5 mg, 0.02 mmol), CuI (9 mg, 0.048 mmol) and triethylamine (0.13 mL, 0.93 mmol) were added to ethyl alcohol (10.0 mL), and stirred for 15 min under the protection of N₂ atmosphere, then the reaction solution was added 1-ethynyl-4-fluorobenzene (86 mg, 0.71 mmol) and stirred at 60 °C for 2 h. After the completion of the reaction, the reaction solution was extracted with water and ethyl acetate, and the organic phase was evaporated to dryness, and purified by column chromatography to afford white solid, i.e. ethyl
2-((4-fluorophenyl)ethynyl)-5,7-dihydro-4H-thieno[2,3-c]pyran-3-carboxylate (81 mg, yield 51%). Ethyl
2-((4-fluorophenyl)ethynyl)-5,7-dihydro-4H-thieno[2,3-c]pyran-3-carboxylate (81 mg, 0.25 mmol), 3.0 mL THF, 3.0 mL methyl alcohol, 1.0 mL water, and lithium hydroxide monohydrate (21 mg, 0.5 mmol) were mixed together, and stirred at 68 °C for 3 h. After the completion of the reaction, the reaction solution was adjusted to acidity with 2M HCl, then extracted with ethyl acetate and water, the organic phase was evaporated to dryness and purified by column chromatography to afford white solid, i.e.
2-((4-fluorophenyl)ethynyl)-5,7-dihydro-4H-thieno[2,3-c]pyran-3-carboxylic acid (70 mg, yield 93%).
2-((4-fluorophenyl)ethynyl)-5,7-dihydro-4H-thieno[2,3-c]pyran-3-carboxylic acid (70 mg, 0.23 mmol), methyl (S)-4-(1-aminoethyl)benzoate (48 mg, 0.26 mmol), HATU (137 mg, 0.36 mmol) and DIEA (65 mg, 0.50 mmol) were dissolved in DMF (2.0 mL) and stirred at rt for 6 h. After the completion of reaction, the reaction mixture was extracted with ethyl acetate and water, the upper organic phase was evaporated to dryness, purified by column chromatography to afford white solid i.e.methyl (S)-4-(1-(2-((4-fluorophenyl)ethynyl)-5,7-dihydro-4H-thieno[2,3-c]pyran-3-carboxam ido)ethyl)benzoate (59 mg, yield 55%). Methyl
(S)-4-(1-(2-((4-fluorophenyl)ethynyl)-5,7-dihydro-4H-thieno[2,3-c]pyran-3-carboxam ido)ethyl)benzoate (59 mg, 0.13 mmol) was dissolved in the solution consisted of 3.0 mL THF, 3.0 mL methyl alcohol, and 1.0 mL water, then lithium hydroxide monohydrate (10 mg, 0.24 mmol) was added, and the reaction solution was stirred at 68 °C for 3 h. After the completion of the reaction, the reaction solution was adjusted to acidity with 2M HCl, extracted with water and ethyl acetate and the organic phase was evaporated to dryness, purified by column chromatography to furnishwhite solid, i.e.final product YJ101(39 mg, yield 66%). ¹H NMR (500 MHz, DMSO) δ 12.80 (s, 1H), 8.88 (d, J = 7.9 Hz, 1H), 7.79 (d, J = 8.2 Hz, 2H), 7.52 (d, J = 8.2 Hz, 2H), 7.42 (m, 2H), 7.23 (t, J = 8.9, 8.9 Hz, 2H), 5.21-5.14 (m, 1H), 4.73 (s, 2H), 3.89-3.81 (m, 2H), 2.63 (d, J = 16.8 Hz, 2H), 1.45 (d, J = 7.0 Hz, 3H).

### Example 1-2. Preparation of (S)-4-(1-(2-((4-fluorophenyl)ethynyl)-5,6-dihydro-4H-cyclopenta[b]thiophene-3-carbo xamido)ethyl)benzoic acid (YJ102) (not part of the invention).

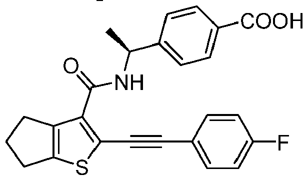

Compound YJ102 (62%, yield of final step) was finally prepared following the same reaction procedure as the preparation of compound YJ101, except using cyclopentanone instead of tetrahydro-4H-pyran-4-one. ¹H NMR (500 MHz, DMSO) δ 8.59 (d, *J =* 7.5 Hz, 1H), 7.81 (d, *J =* 7.6 Hz, 2H), 7.52 (d, *J =* 7.7 Hz, 2H), 7.45-7.43 (m, 2H), 7.23 (t, *J =* 8.4, 8.8 Hz, 2H), 5.18-5.13 (m, 1H), 1.80 (m, 6H), 1.45 (d, *J =* 6.8 Hz, 3H).

### Example 1-3. Preparation of 4-((1S)-1-(6-ethyl-2-((4-fluorophenyl)ethynyl)-4,5,6,7-tetrahydrobenzo[b]thiophene-3 -carboxamido)ethyl)benzoic acid (YJ103) (not part of the invention).

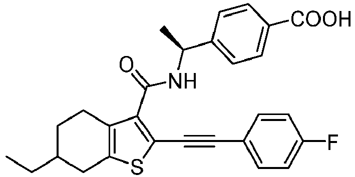

Compound YJ103 (64%, yield of final step) was finally prepared following the same reaction procedure as the preparation of compound YJ101, except using 4-ethylcyclohexanone instead of tetrahydro-4H-pyran-4-one. ¹H NMR (500 MHz, DMSO) δ 12.79 (s, 1H), 8.86 (d, *J* = 7.9 Hz, 1H), 7.79 (t, *J* = 8.6, 8.9 Hz, 2H), 7.52 (d, *J* = 6.9 Hz, 2H), 7.41-7.35 (m, 2H), 7.24-7.19 (m, 2H), 5.22-5.14 (m, 1H), 2.87 (d, *J* = 16.7 Hz, 1H), 2.64-2.58 (m, 1H), 2.36-2.29 (m, 1H), 1.88 (d, *J* = 6.8 Hz, 1H), 1.65 (d, *J* = 5.3 Hz, 1H), 1.44 (d, *J* = 7.0 Hz, 3H), 1.41-1.36 (m, 2H), 1.35-1.31 (m, 1H), 1.24 (s, 1H), 0.94 (t, *J* = 7.4, 7.4 Hz, 3H).

### Example 1-4. Preparation of (S)-4-(1-(2-((4-fluorophenyl)ethynyl)-5,6,7,8-tetrahydro-4H-cyclohepta[b]thiophene-3 -carboxamido)ethyl)benzoic acid (YJ104) (not part of the invention.

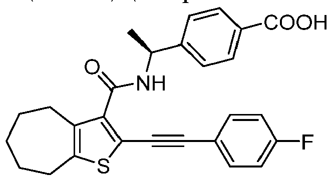

Compound YJ104 (61%, yield of final step) was finally prepared following the same reaction procedure as the preparation of compound YJ101, except using cycloheptanone instead of tetrahydro-4H-pyran-4-one. ¹H NMR (500 MHz, DMSO) δ 12.79 (s, 1H), 9.01 (d, *J* = 8.0 Hz, 1H), 7.77 (d, *J* = 8.0 Hz, 2H), 7.51 (d, *J* = 8.1 Hz, 2H), 7.40-7.33 (m, 2H), 7.21 (t, *J* = 8.8, 8.6 Hz, 2H), 5.26-5.13 (m, 1H), 2.86-2.75 (m, 2H), 2.59 (m, 2H), 1.83 (s, 2H), 1.57 (d, *J* = 39.5 Hz, 4H), 1.42 (d, *J* = 7.0 Hz, 3H).

### Example 1-5. Preparation of (S)-4-(1-(2-((4-fluorophenyl)ethynyl)-5,5-dimethyl-5,7-dihydro-4H-thieno[2,3-c]pyra n-3-carboxamido)ethyl)benzoic acid (YJ105).

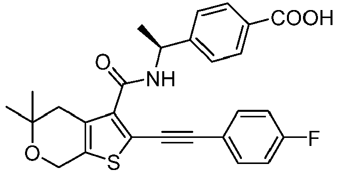

Compound YJ105 (70%, yield of final step) was finally prepared following the same reaction procedure as the preparation of compound YJ101, except using tetrahydro-2,2-dimethyl-4H-pyran-4-one instead of tetrahydro-4H-pyran-4-one. ¹H NMR (400 MHz, DMSO) δ 12.80 (s, 1H), 8.88 (d, *J* = 7.8 Hz, 1H), 7.78 (d, *J* = 8.1 Hz, 2H), 7.54-7.38 (m, 4H), 7.23 (t, *J* = 11.1, 10.7 Hz, 2H), 5.23-5.07 (m, 1H), 4.71 (s, 2H), 2.53 (s, 2H), 1.44 (d, *J* = 7.0 Hz, 3H), 1.21 (s, 6H).

### Example 1-6. Preparation of (S)-4-(1-(2-(4-fluorophenethyl)-4,7-dihydro-5H-thieno[2,3-c]pyran-3-carboxamido)et hyl)benzoic acid (YJ106).

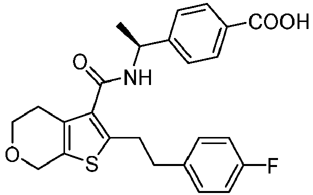

Tetrahydro-4H-pyran-4-one (2.00 g, 20.0 mmol), ethyl cyanoacetate (2.50 g, 22.0 mmol) and sulphur (704 mg, 22.0 mmol) were dissolved in ethyl alcohol (30.0 mL), then the solution was added morpholine (1.74 g, 20.0 mmol) and stirred and reacted at 50 °C overnight. The reaction was monitored by TLC. After the completion of the reaction, the reaction solution was extracted with ethyl acetate and water and the upper organic phase was obtained and evaporated to dryness, purified by column chromatography to afford pale yellow solid, i.e. ethyl
2-amino-5,7-dihydro-4H-thieno[2,3-c]pyran-3-carboxylate (4.42 g, yield 97%). Ethyl 2-amino-5,7-dihydro-4H-thieno[2,3-c]pyran-3-carboxylate (450 mg, 2.0 mmol) was dissolved in 1.5 M HCl(10.0 mL), and stirred for 20 min at room temperature, then cooled to 0 °C in ice bath. NaNO₂ (207 mg, 3.0 mmol) was added to the solution and the mixture was stirred for 30 min under ice bath. Subsequently, the reaction solution was added KI (830 mg, 5.0 mmol) in portions, and continued to react at 0 °C for 45 min. After the completion of the reaction, the reaction solution was extracted with water and ethyl acetate, and the organic phase was evaporated to dryness and purified by column chromatography to afford pale yellow solid, i.e. ethyl 2-iodo-5,7-dihydro-4H-thieno[2,3-c]pyran-3-carboxylate (190 mg, yield 28%).

Ethyl 2-iodo-5,7-dihydro-4H-thieno[2,3-c]pyran-3-carboxylate (160 mg, 0.48 mmol), 1-ethynyl-4-fluorobenzene (86 mg, 0.71 mmol), 10% Pd/C (5 mg, 0.048 mmol), PPh₃ (5 mg, 0.02 mmol), CuI (9 mg, 0.048 mmol), and triethylamine (0.13 mL, 0.93 mmol) were added to ethyl alcohol (10.0 mL), and stirred for 15 min under the protection of N₂ atmosphere. Then 1-ethynyl-4-fluorobenzene (86 mg, 0.71 mmol) was added and continue to stir at 60 °C for 2 h. After the completion of the reaction, the reaction solution was extracted with water and ethyl acetate, the organic phases were evaporated to dryness, and purified by column chromatography to afford white solid, i.e. ethyl
2-((4-fluorophenyl)ethynyl)-5,7-dihydro-4H-thieno[2,3-c]pyran-3-carboxylate (91 mg, yield 57%). Ethyl
2-((4-fluorophenyl)ethynyl)-5,7-dihydro-4H-thieno[2,3-c]pyran-3-carboxylate (80 mg, 0.24 mmol) and 10% Pd/C (10 mg) were added to absolute ethyl alcohol (10 mL), the reaction was bubbled with H₂ and stirred at rt overnight. After the completion of the reaction, the reaction solution was filtered, the filtrate was obtained and evaporated under reduced pressure to remove the solvent, purified by column chromatography to afford white solid, i.e. ethyl
2-(4-fluorophenethyl)-4,7-dihydro-5H-thieno[2,3-c]pyran-3-carboxylate (82 mg, 98% yield). Ethyl 2-(4-fluorophenethyl)-5,7-dihydro-4H-thieno[2,3-c]pyran-3-carboxylate (80 mg, 0.24 mmol), 3.0 mL THF, 3.0 mL methyl alcohol, 1.0 mL water, and lithium hydroxide monohydrate (21 mg, 0.5 mmol) were mixed together, and stirred at 68 °C for 3 h. After the completion of the reaction, the reaction solution was adjusted to acidity with 2M HCl, then extracted with ethyl acetate and water, the organic phase was evaporated to dryness and purified by column chromatography to afford white solid, i.e. 2-(4-fluorophenethyl)-5,7-dihydro-4H-thieno[2,3-c]pyran-3-carboxylic acid(64 mg, yield 85%).
2-(4-fluorophenethyl)-5,7-dihydro-4H-thieno[2,3-c]pyran-3-carboxylic acid (64 mg, 0.21 mmol), methyl (S)-4-(1-aminoethyl)benzoate (48 mg, 0.26 mmol), HATU (137 mg, 0.36 mmol) and DIEA (65 mg, 0.50 mmol) were dissolved in DMF (2.0 mL) and stirred at rt for 6 h. After the completion of reaction, the reaction solution was extracted with ethyl acetate and water, the upper organic phase was evaporated to dryness, purified by column chromatography to afford white solid i.e.methyl (S)-4-(1-(2-(4--fluorophenethyl)-5,7-dihydro-4H-thieno[2,3-c]pyran-3-carboxamido)et hyl)benzoate (62 mg, yield 57%). Methyl
(S)-4-(1-(2-(4-fluorophenethyl)-5,7-dihydro-4H-thieno[2,3-c]pyran-3-carboxamido)et hyl)benzoate (61 mg, 0.13 mmol) was dissolved in the solution consisted of 3.0 mL THF, 3.0 mL methyl alcohol, and 1.0 mL water, then lithium hydroxide monohydrate (10 mg, 0.24 mmol) was added, and the reaction solution was stirred at 68 °C for 3 h.

After the completion of the reaction, the reaction solution was adjusted to acidity with 2M HCl, extracted with water and ethyl acetate and the organic phase was evaporated to dryness, purified by column chromatography to afford white solid, i.e.final product YJ106 (41 mg, yield 69%). ¹H NMR (500 MHz, DMSO) δ 12.86 (s, 1H), 8.68 (d, J = 7.8 Hz, 1H), 7.89 (d, J = 7.8 Hz, 2H), 7.49 (d, J = 7.9 Hz, 2H), 7.10-7.01 (m, 4H), 5.16 (s, 1H), 4.65 (s, 2H), 3.82 (s, 2H), 3.06-2.96 (m, 2H), 2.75 (t, J = 7.6, 7.1 Hz, 2H), 2.60 (s, 2H), 1.43 (d, J = 6.9 Hz, 3H).

### Example 1-7. Preparation of (S)-4-(1-(2-(4-fluorophenethyl)-5,6,7,8-tetrahydro-4H-cyclohepta[b]thiophene-3-carb oxamido)ethyl)benzoic acid (YJ107) (not part of the invention).

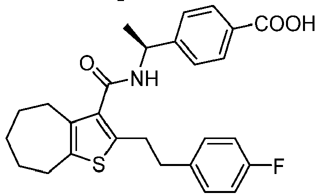

Compound YJ107 (63%, yield of final step) was finally prepared following the same reaction procedure as the synthesis of compound YJ106, except using cycloheptanone instead of tetrahydro-4H-pyran-4-one. ¹H NMR (500 MHz, DMSO) δ 12.81 (s, 1H), 8.81 (d, *J* = 7.9 Hz, 1H), 7.88 (d, *J* = 7.9 Hz, 2H), 7.49 (d, *J* = 8.0 Hz, 2H), 7.10-6.97 (m, 4H), 5.23-5.08 (m, 1H), 2.89-2.64 (m, 8H), 1.80 (s, 2H), 1.56 (d, *J* = 25.3 Hz, 4H), 1.42 (d, *J* = 6.9 Hz, 3H).

### Example 1-8. Preparation of (S)-4-(1-(2-(4-fluorophenethyl)-4,5,6,7-tetrahydrobenzo[b]thiophene-3-carboxamido)e thyl)benzoic acid (YJ108) (not part of the invention).

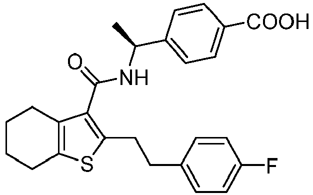

Compound YJ108 (71%, yield of final step) was finally prepared following the same reaction procedure as the synthesis of compound YJ106, except using cyclohexanone instead of tetrahydro-4H-pyran-4-one. ¹H NMR (500 MHz, DMSO) δ 12.86 (s, 1H), 8.67 (d, *J* = 8.1 Hz, 1H), 7.89 (d, *J* = 8.3 Hz, 2H), 7.50 (d, *J* = 8.3 Hz, 2H), 7.10-7.02 (m, 4H), 5.19-5.14 (m, 1H), 2.97-2.91 (m, 2H), 2.74 (t, *J =* 7.7 Hz, 2H), 2.64 (s, 2H), 2.47 (t, *J =* 8.2, 7.2 Hz, 2H), 1.77-1.73 (m, 2H), 1.72-1.67 (m, 2H), 1.42 (d, *J* = 7.1 Hz, 3H).

### Example 1-9. Preparation of (S)-4-(1-(2-(4-fluorophenethyl)-5,6-dihydro-4H-cyclopenta[b]thiophene-3-carboxami do)ethyl)benzoic acid (YJ109) (not part of the invention.

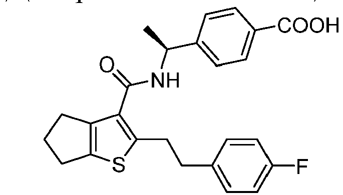

Compound YJ109 (60%, yield of final step) was finally prepared following the same chemical reaction procedure as the preparation of compound YJ106, except using cyclopentanone instead of tetrahydro-4H-pyran-4-one. ¹H NMR (500 MHz, DMSO) δ 12.84 (s, 1H), 8.35 (d, *J* = 7.9 Hz, 1H), 7.90 (d, *J* = 8.3 Hz, 2H), 7.49 (d, *J* = 8.2 Hz, 2H), 7.13-7.10 (m, 2H), 7.06-7.02 (m, 2H), 5.16-5.11 (m, 1H), 3.11-3.06 (m, 2H), 2.80-2.75 (m, 6H), 2.38-2.34 (m, 2H), 1.43 (d, *J =* 7.1 Hz, 3H).

### Example 1-10. Preparation of (S)-4-(1-(2-(4-fluorophenethyl)-5,5-dimethyl-5,7-dihydro-4H-thieno[2,3-c]pyran-3-ca rboxamido)ethyl)benzoic acid (YJ110).

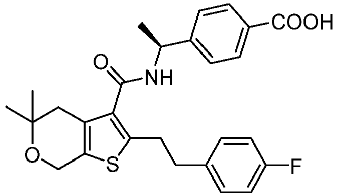

Compound YJ110 (67%, yield of final step) was finally prepared following the same chemical reaction procedure as the preparation of compound YJ106, except using tetrahydro-2,2-dimethyl-4H-pyran-4-one instead of tetrahydro-4H-pyran-4-one. ¹H NMR (400 MHz, DMSO) δ 12.84 (s, 1H), 8.68 (d, *J* = 8.1 Hz, 1H), 7.89 (d, *J* = 8.2 Hz, 2H), 7.49 (d, *J =* 8.2 Hz, 2H), 7.09-7.00 (m, 4H), 5.19-5.12 (m, 1H), 4.63 (s, 2H), 3.05-2.96 (m, 2H), 2.75 (t, *J* = 10.0, 10.0 Hz, 2H), 2.47 (s, 2H), 1.43 (d, *J* = 7.0 Hz, 3H), 1.19 (s, 6H).

### Example 1-11. Preparation of (S)-4-(1-(2-(4-(trifluoromethyl)phenethyl)-5,7-dihydro-4H-thieno[2,3-c]pyran-3-carbo xamido)ethyl)benzoic acid (YJ111).

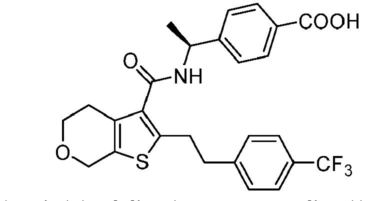

Compound YJ111 (73%, yield of final step) was finally prepared following the same chemical reaction procedure as the preparation of compound YJ106, except using 1-ethynyl-4-(trifluoromethyl) benzene instead of 1-ethynyl-4-fluorobenzene . ¹H NMR (500 MHz, DMSO) δ 12.87 (s, 1H), 8.70 (d, *J* = 8.0 Hz, 1H), 7.90 (d, *J* = 8.0 Hz, 2H), 7.58 (d, *J* = 8.0 Hz, 2H), 7.50 (d, *J* = 8.0 Hz, 2H), 7.28 (d, *J* = 7.9 Hz, 2H), 5.21-5.14 (m, 1H), 4.66 (s, 2H), 3.84 (d, *J* = 5.6 Hz, 2H), 3.09-3.05 (m, 2H), 2.88-2.85 (m, 2H), 2.62 (s, 2H), 1.43 (d, *J* = 7.0 Hz, 3H).

### Example 1-12. Preparation of (S)-4-(1-(2-(3-(trifluoromethyl)phenethyl)-5,7-dihydro-4H-thieno[2,3-c]pyran-3-carbo xamido)ethyl)benzoic acid (YJ112).

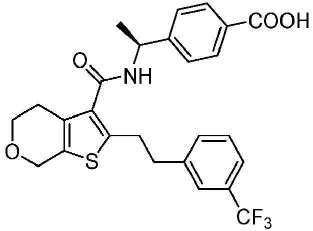

Compound YJ112 (75%, yield of final step) was finally prepared following the same chemical reaction procedure as the preparation of compound YJ106, except using 1-ethynyl-3-(trifluoromethyl) benzene instead of 1-ethynyl-4-fluorobenzene. ¹H NMR (500 MHz, DMSO) δ 12.77 (s, 1H), 8.70 (d, *J* = 8.1 Hz, 1H), 7.88 (d, *J* = 6.9 Hz, 2H), 7.55 (d, *J* = 7.3 Hz, 1H), 7.51-7.46 (m, 4H), 7.37 (d, *J* = 7.3 Hz, 1H), 5.22-5.12 (m, 1H), 4.65 (s, 2H), 3.83 (s, 2H), 3.14-3.04 (m, 2H), 2.93-2.85 (m, 2H), 2.61 (d, *J* = 4.4 Hz, 2H), 1.43 (d, *J* = 6.9 Hz, 3H).

### Example 1-13. Preparation of (S)-4-(1-(2-(3-fluorophenethyl)-4,7-dihydro-5H-thieno[2,3-c]pyran-3-carboxamido)et hyl)benzoic acid (YJ113).

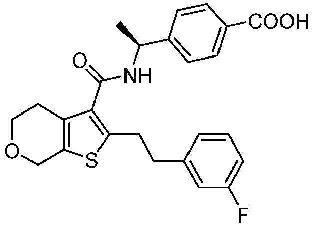

Compound YJ113 (68%, yield of final step) was finally prepared following the same chemical reaction procedure as the preparation of compound YJ106, except using 1-ethynyl-3-fluorobenzene instead of 1-ethynyl-4-fluorobenzene. ¹H NMR (500 MHz, DMSO) δ 12.82 (s, 1H), 8.69 (d, *J* = 7.4 Hz, 1H), 7.89 (d, *J* = 7.8 Hz, 2H), 7.50 (d, *J* = 6.7 Hz, 2H), 7.27 (d, *J* = 7.5 Hz, 1H), 7.04-6.88 (m, 3H), 5.17 (s, 1H), 4.66 (s, 2H), 3.83 (s, 2H), 3.06 (d, *J* = 8.0 Hz, 2H), 2.80 (s, 2H), 2.61 (s, 2H), 1.43 (d, *J* = 6.5 Hz, 3H).

### Example 1-14. Preparation of (S)-4-(1-(2-(4-fluorobenzyl)-5,7-dihydro-4H-thieno[2,3-c]pyran-3-carboxamido)ethyl) benzoic acid (YJ114).

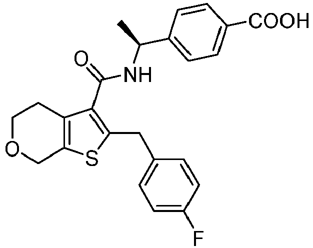

Tetrahydro-4H-pyran-4-one (2.00 g, 20.0 mmol), ethyl cyanoacetate (2.50 g, 22.0 mmol) and sulphur (704 mg, 22.0 mmol) were dissolved in ethyl alcohol (30.0 mL), then the solution was added morpholine (1.74 g, 20.0 mmol), and stirred at 50 °C overnight. The reaction was monitored by TLC. After the completion of the reaction, the reaction solution was extracted with ethyl acetate and water, and the upper organic phase was evaporated to dryness, purified by column chromatography to afford pale yellow solid, i.e. ethyl 2-amino-5,7-dihydro-4H-thieno[2,3-c]pyran-3-carboxylate (4.29 g, yield 94%). Ethyl 2-amino-5,7-dihydro-4H-thieno[2,3-c]pyran-3-carboxylate (900 mg, 4.0 mmol) was dissolved in 1.5 M HCl(20.0 mL), and stirred for 20 min at room temperature, then cooled to 0 °C in ice bath. NaNO₂ (414 mg, 6.0 mmol) was added to the solution and the reaction solution was stirred for 30 min under ice bath. Subsequently, KI (830 mg, 5.0 mmol) was added in portions, and continue to react at 0°C for 45 min. After the completion of the reaction, the reaction solution was extracted with ethyl acetate and water, and the organic phase was evaporated to dryness and purified by column chromatography to afford pale yellow solid, i.e. ethyl 2-iodo-5,7-dihydro-4H-thieno[2,3-c]pyran-3-carboxylate (324 mg, yield 24%)*. n*-Butyl lithium (1.6M in hexane) was added into 10.0 mL diethyl ether at -78 °C, then ethyl 2-iodo-5,7-dihydro-4H-thieno[2,3-c]pyran-3-carboxylate (200 mg, 0.62 mmol) was added in portions into the solution at -78 °C, keeping the temperature constant and the reaction was stirred for 1.5 h. After that, the solution was added p-fluorobenzaldehyde (85 mg, 0.68 mmol)dropwiseand then stirred at -78 °C for 1 h, after that allowed to warm to 0 °C and stirred at 0 °C for another 1 h. After the completion of the reaction, the reaction solution was added saturated ammonium chloride aqueous solution (10 mL) and extracted with ethyl acetate. The upper organic phase was evaporated to dryness. Then the product was added into 10 mL dichloromethane at 0 °C without further purification and dissolved, keeping the tmepurature constant, then triethylsilane (0.41 mL, 2.48 mmol) was added quickly to the solution and followed by trifluoroacetic acid (0.47 mL, 6.20 mmol) dropwise. After the reaction solution was stirred and reacted at 0 °C for 30 min, the solvent was evaporated to dryness, then dissolved in trichloromethane and washed with 5% NaHCO₃ aqueous solution. The organic phase was obtained, washed with saturated NaCl solution, then evaporated to dryness and purified by column chromatography to afford white solid, i.e. ethyl
2-(4-fluorobenzyl)-5,7-dihydro-4H-thieno[2,3-c]pyran-3-carboxylate(172 mg, 91% yield of two steps). Ethyl
2-(4-fluorobenzyl)-5,7-dihydro-4H-thieno[2,3-c]pyran-3-carboxylate (80 mg, 0.25 mmol), 3.0 mL THF, 3.0 mL methyl alcohol, 1.0 mL water, and lithium hydroxide monohydrate (21 mg, 0.5 mmol) were mixed together, and stirred at 68 °C for 3 h. After the completion of the reaction, the reaction solution was adjusted to acidity with 2M HCl, then extracted with ethyl acetate and water, the organic phase was evaporated to dryness and purified by column chromatography to afford white solid, i.e. 2-(4-fluorobenzyl)-5,7-dihydro-4H-thieno[2,3-c]pyran-3-carboxylic acid (69 mg, yield 96%). 2-(4-Fluorobenzyl)-5,7-dihydro-4H-thieno[2,3-c]pyran-3-carboxylic acid (64 mg, 0.22 mmol), methyl (S)-4-(1-aminoethyl)benzoate (48 mg, 0.26 mmol), HATU (137 mg, 0.36 mmol) and DIEA (65 mg, 0.50 mmol) were dissolved in DMF (2.0 mL) and stirred at rt for 6 h. After the completion of reaction, the reaction solution was extracted with ethyl acetate and water, the upper organic phase was evaporated to dryness, purified by column chromatography to afford white solid i.e.methyl (S)-4-(1-(2-(4-fluorobenzyl)-5,7-dihydro-4H-thieno[2,3-c]pyran-3-carboxamido)ethyl) benzoate (62 mg, yield 62%). Methyl
(S)-4-(1-(2-(4-fluorobenzyl)-5,7-dihydro-4H-thieno[2,3-c]pyran-3-carboxamido)ethyl) benzoate (60 mg,0.13 mmol) was dissolved in the solution consisted of 3.0 mL THF, 3.0 mL methyl alcohol, and 1.0 mL water, then lithium hydroxide monohydrate (10 mg, 0.24 mmol) was added, and the reaction solution was stirred and reacted at 68 °C for 3 h. After the completion of the reaction, the reaction solution was adjusted to acidity with 2M HCl, extracted with water and ethyl acetate, and the organic phase was evaporated to dryness, and purified by column chromatography to afford white solid, i.e. the final product YJ114 (35 mg,61% yield). ¹H NMR (500 MHz, DMSO) δ 12.79 (s, 1H), 8.73 (d, J = 7.7 Hz, 1H), 7.90 (d, J = 7.5 Hz, 2H), 7.47 (d, J = 7.7 Hz, 2H), 7.18 (t, J = 6.3, 6.4 Hz, 2H), 7.05 (t, J = 8.5, 8.2 Hz, 2H), 5.17-5.12 (m, 1H), 4.63 (s, 2H), 4.16-4.06 (m, 2H), 3.82 (s, 2H), 2.62 (s, 2H), 1.42 (d, J = 7.4 Hz, 3H).

### Example 1-15. Preparation of (S)-4-(1-(2-(4-(trifluoromethyl)benzyl)-5,7-dihydro-4H-thieno[2,3-c]pyran-3-carboxa mido)ethyl)benzoic acid (YJ115).

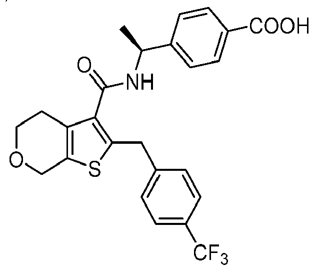

Compound YJ115 (73%, yield of final step) was finally prepared following the same chemical reaction procedure as the preparation of compound YJ114, except using p-(trifluoromethyl)benzaldehyde instead of p-fluorobenzaldehyde. ¹H NMR (400 MHz, DMSO) δ 12.85 (s, 1H), 8.73 (d, *J* = 7.6 Hz, 1H), 7.89 (d, *J* = 7.8 Hz, 2H), 7.57 (d, *J* = 7.7 Hz, 2H), 7.46 (d, *J* = 7.9 Hz, 2H), 7.35 (d, *J* = 7.8 Hz, 2H), 5.20-5.09 (m, 1H), 4.64 (s, 2H), 4.29-4.16 (m, 2H), 3.82 (s, 2H), 2.62 (s, 2H), 1.41 (d, *J* = 6.8 Hz, 3H).

### Example 1-16. Preparation of (S)-4-(1-(2-(3-(trifluoromethyl)benzyl)-5,7-dihydro-4H-thieno[2,3-c]pyran-3-carboxa mido)ethyl)benzoic acid (YJ116).

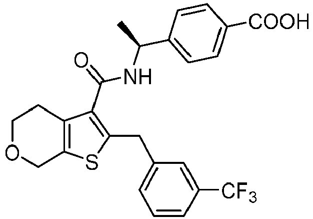

Compound YJ116 (60%, yield of final step) was finally prepared following the same chemical reaction procedure as the preparation of compound YJ114, except using 3-(trifluoromethyl) benzaldehyde instead of p-fluorobenzaldehyde. ¹H NMR (400 MHz, DMSO) δ 12.83 (s, 1H), 8.77 (d, *J* = 7.9 Hz, 1H), 7.88 (d, *J* = 7.9 Hz, 2H), 7.56 (d, *J =* 5.9 Hz, 2H), 7.50-7.45 (m, 4H), 5.17-5.11 (m, 1H), 4.64 (s, 2H), 4.28-4.18 (m, 2H), 3.82 (t, *J* = 6.5, 6.4 Hz, 2H), 2.65 (d, *J* = 17.3 Hz, 2H), 1.41 (d, *J* = 6.9 Hz, 3H).

### Example 1-17. Preparation of (R)-4-(1-(2-(4-fluorobenzyl)-5,7-dihydro-4H-thieno[2,3-c]pyran-3-carboxamido)ethyl benzoic acid (YJ117).

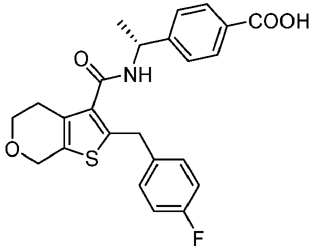

Compound YJ117 (68%, yield of final step) was finally prepared following the same chemical reaction procedure as the preparation of compound YJ114, except using methyl (R)-4-(1-aminoethyl)benzoate instead of methyl (S)-4-(1-aminoethyl)benzoate. ¹H NMR (500 MHz, DMSO) δ 12.84 (s, 1H), 8.73 (d, *J* = 7.9 Hz, 1H), 7.90 (d, *J* = 7.7 Hz, 2H), 7.48 (d, *J =* 7.8 Hz, 2H), 7.18 (t, *J* = 6.7, 6.1 Hz, 2H), 7.05 (t, *J =* 8.5, 8.5 Hz, 2H), 5.19-5.11 (m, 1H), 4.63 (s, 2H), 4.16-4.06 (m, 2H), 3.82 (s, 2H), 2.62 (s, 2H), 1.42 (d, *J =* 6.9 Hz, 3H).

### Example 1-18. Preparation of (R)-4-(1-(2-(4-(trifluoromethyl)benzyl)-5,7-dihydro-4H-thieno[2,3-c]pyran-3-carboxa mido)ethyl)benzoic acid (YJ118).

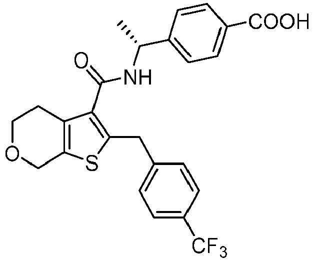

Compound YJ118 (61%, yield of final step) was finally prepared following the same chemical reaction procedure as the synthesis of compound YJ114, except using *p*-trifluoromethylbenzaldehyde instead of *p*-fluorobenzaldehyde and in the subsequent steps using methyl (R)-4-(1-aminoethyl)benzoate instead of methyl (S)-4-(1-aminoethyl)benzoate. ¹H NMR (500 MHz, DMSO) δ 12.82 (s, 1H), 8.73 (d, *J* = 8.0 Hz, 1H), 7.90 (d, *J* = 8.1 Hz, 2H), 7.57 (d, *J* = 8.0 Hz, 2H), 7.47 (d, *J* = 8.1 Hz, 2H), 7.36 (d, *J =* 7.9 Hz, 2H), 5.18-5.12 (m, 1H), 4.65 (s, 2H), 4.29-4.17 (m, 2H), 3.84-3.80 (m, 2H), 2.63 (d, *J* = 5.3 Hz, 2H), 1.41 (d, *J* = 7.0 Hz, 3H).

### Example 1-19. Preparation of (R)-4-(1-(2-(3-(trifluoromethyl)benzyl)-5,7-dihydro-4H-thieno[2,3-c]pyran-3-carboxa mido)ethyl)benzoic acid (YJ119).

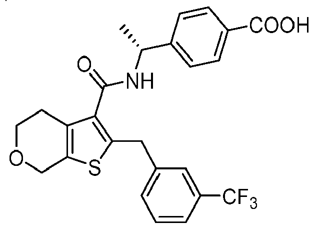

Compound YJ119 (70%, yield of final step) was finally prepared following the same chemical reaction procedure as the synthesis of compound YJ114, except using 3-trifluoromethylbenzaldehyde instead of p-fluorobenzaldehyde and in the subsequent steps using methyl (R)-4-(1-aminoethyl)benzoate instead of methyl (S)-4-(1-aminoethyl)benzoate. ¹H NMR (500 MHz, DMSO) δ 12.84 (s, 1H), 8.77 (d, *J* = 8.0 Hz, 1H), 7.89 (d, *J =* 8.0 Hz, 2H), 7.57 (d, *J =* 6.3 Hz, 2H), 7.48 (t, *J =* 6.6, 6.8 Hz, 4H), 5.18-5.12 (m, 1H), 4.64 (s, 2H), 4.27-4.20 (m, 2H), 3.83 (t, *J =* 5.3, 5.4 Hz, 2H), 2.63 (s, 2H), 1.42 (d, *J* = 7.0 Hz, 3H).

### Example 1-20. Preparation of 4-((2-(4-fluorobenzyl)-4,7-dihydro-5H-thieno[2,3-c]pyran-3-carboxamido)methyl)ben zoic acid (YJ120).

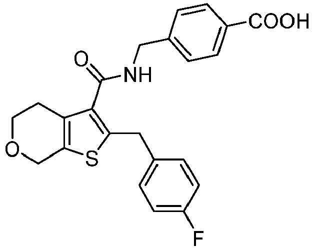

Compound YJ120 (90%, yield of final step) was finally prepared following the same chemical reaction procedure as the synthesis of compound YJ114, except using methyl 4-(aminomethyl)benzoate hydrochloride instead of methyl (S)-4-(1-aminoethyl)benzoate. ¹H NMR (500 MHz, DMSO) δ 12.91 (s, 1H), 8.75 (t, *J* = 6.1 Hz, 1H), 7.89 (d, *J* = 6.7 Hz, 2H), 7.39 (d, *J* = 8.3 Hz, 2H), 7.22 (dd, *J* = 8.7, 5.6 Hz, 2H), 7.08 (t, *J* = 10.4 Hz, 2H), 4.63 (s, 2H), 4.48 (d, *J* = 6.0 Hz, 2H), 4.17 (s, 2H), 3.82 (t, *J* = 5.5 Hz, 2H), 2.65 (t, *J* = 5.5 Hz, 2H).

### Example 1-21. Preparation of (S)-4-(1-(2-((4-(trifluoromethyl)benzyl)amino)-5,7-dihydro-4H-thieno[2,3-c]pyran-3-carboxamido)ethyl)benzoic acid (YJ121).

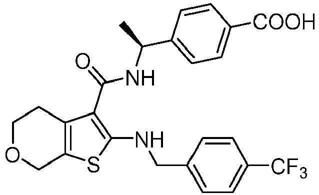

Tetrahydro-4H-pyran-4-one (2.00 g, 20.0 mmol), ethyl cyanoacetate (2.50 g, 22.0 mmol) and sulphur (704 mg, 22.0 mmol) were dissolved in ethyl alcohol (30.0 mL), then the solution was added morpholine (1.74 g, 20.0 mmol) , and stirred at 50 °C overnight. The reaction was monitored by TLC. After the completion of the reaction, the reaction solution was extracted with ethyl acetate and water and the upper organic phase was evaporated to dryness, purified by column chromatography to afford pale yellow solid, i.e. ethyl 2-amino-5,7-dihydro-4H-thieno[2,3-c]pyran-3-carboxylate (4.20 g, yield 92%). Ethyl 2-amino-5,7-dihydro-4H-thieno[2,3-c]pyran-3-carboxylate (260 mg, 1.14 mmol), 4-(trifluoromethyl)benzyl bromide (215 mg, 1.14 mmol) and Cs₂CO₃ (929 mg, 2.85 mmol) was added to acetone (15.0 mL) and the reaction mixture was stirred at 60 °C overnight. After the completion of the reaction, the solvent was evaporated to dryness and purified by column chromatography to afford white solid, i.e. ethyl
2-((4-(trifluoromethyl)benzyl)amino)-5,7-dihydro-4H-thieno[2,3-c]pyran-3-carboxylat e(236 mg, yield 62%). Ethyl
2-((4-(trifluoromethyl)benzyl)amino)-5,7-dihydro-4H-thieno[2,3-c]pyran-3-carboxylat e (116 mg, 0.3 mmol), 3.0 mL THF, 3.0 mL methyl alcohol, 1.0 mL water, and lithium hydroxide monohydrate (25 mg, 0.6 mmol) were mixed together, and stirred at 68 °C for 3 h. After the completion of the reaction, the reaction solution was adjusted to acidity with 2M HCl, then extracted with ethyl acetate and water, the organic phase was evaporated to dryness and purified by column chromatography to afford white solid, i.e.
2-((4-(trifluoromethyl)benzyl)amino)-5,7-dihydro-4H-thieno[2,3-c]pyran-3-carboxylic acid(99 mg, yield
92%).2-((4-(trifluoromethyl)benzyl)amino)-5,7-dihydro-4H-thieno[2,3-c]pyran-3-carb oxylic acid (82 mg, 0.23 mmol), methyl (S)-4-(1-aminoethyl)benzoate (48 mg, 0.26 mmol), HATU (137 mg, 0.36 mmol), and DIEA (65 mg, 0.50 mmol) were dissolved in DMF (2.0 mL) and stirred at rt for 6 h. After the completion of reaction, the reaction solution was extracted with ethyl acetate and water, the upper organic phase was evaporated to dryness, purified by column chromatography to afford white solid i.e.methyl
(S)-4-(1-(2-((4-(trifluoromethyl)benzyl)amino)-5,7-dihydro-4H-thieno[2,3-c]pyran-3-carboxamido)ethyl)benzoate (72 mg, yield 63%). Methyl
(S)-4-(1-(2-((4-(trifluoromethyl)benzyl)amino)-4,7-dihydro-5H-thieno[2,3-c]pyran-3-carboxamido)ethyl)benzoate (62 mg,0.12 mmol) was dissolved in the solution consisted of 3.0 mL THF, 3.0 mL methyl alcohol, and 1.0 mL water, then lithium hydroxide monohydrate (10 mg, 0.24 mmol) was added, and the reaction mixture was stirred and reacted at 68 °C for 3 h. After the completion of the reaction, the reaction solution was adjusted to acidiity with 2M HCl, extracted with water and ethyl acetate, and the organic phase was evaporated to dryness, and purified by column chromatography to afford white solid, i.e. the final product YJ121 (35 mg,58% yield). 1H NMR (400 MHz, DMSO) δ 12.85 (s, 1H), 8.73 (d, J = 8.0 Hz, 1H), 7.89 (d, J = 7.9 Hz, 2H), 7.56 (d, J = 7.9 Hz, 3H), 7.51 (d, J = 8.0 Hz, 1H), 7.43 (d, J = 7.9 Hz, 3H), 5.20-5.13 (m, 7.0 Hz, 1H), 4.53 (s, 2H), 4.25-4.14 (m, 4H), 3.87-3.76 (m, 2H), 1.43 (d, J = 7.0 Hz, 3H)

### Example 1-22. Preparation of (S)-4-(1-(2-(4-methoxyphenethyl)-4,7-dihydro-5H-thieno[2,3-c]pyran-3-carboxamido) ethyl)benzoic acid (YJ122).

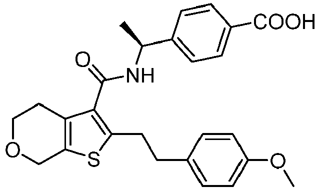

Compound YJ122 (80%, yield of final step) was finally prepared following the same chemical reaction procedure as the preparation of compound YJ106, except using 1-ethynyl-4-(methoxy) benzene instead of 1-ethynyl-4-fluorobenzene. ¹H NMR (500 MHz, DMSO) δ 12.85 (s, 1H), 8.70 (d, *J* = 8.1 Hz, 1H), 7.90 (d, *J* = 8.2 Hz, 2H), 7.50 (d, *J* = 8.2 Hz, 2H), 6.96 (d, *J* = 8.5 Hz, 2H), 6.78 (d, *J* = 8.5 Hz, 2H), 5.22 - 5.12 (m, 1H), 4.66 (s, 2H), 3.83 (t, *J* = 5.9 Hz, 2H), 3.71 (s, 3H), 2.98 (m, *J* = 14.8, 7.1 Hz, 2H), 2.70 (t, *J =* 7.9 Hz, 2H), 2.60 (s, 2H), 1.43 (d, *J* = 7.1 Hz, 3H).

### Example 1-23. Preparation of (S)-4-(1-(2-(3-fluorobenzyl)-4,7-dihydro-5H-thieno[2,3-c]pyran-3-carboxamido)ethyl) benzoic acid (YJ123).

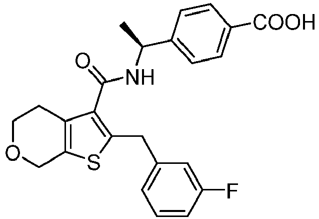

Compound YJ123 (82%, yield of final step) was finally prepared following the same chemical reaction procedure as the preparation of compound YJ114, except using 3-fluorobenzaldehyde instead of p-fluorobenzaldehyde. ¹H NMR (500 MHz, DMSO) δ 12.83 (s, 1H), 8.78 (d, *J* = 7.9 Hz, 1H), 7.89 (d, *J* = 8.1 Hz, 2H), 7.47 (d, *J* = 8.1 Hz, 2H), 7.28 (s, 1H), 7.00 (m, *J* = 13.4, 7.3 Hz, 3H), 5.21 - 5.10 (m, 1H), 4.64 (s, 2H), 4.15 (m, *J* = 31.4, 15.5 Hz, 2H), 3.83 (s, 2H), 2.63 (m, 2H), 1.42 (d, *J* = 7.0 Hz, 3H).

### Example 1-24. Preparation of (S)-4-(1-(2-(4-chlorobenzyl)-4,7-dihydro-5H-thieno[2,3-c]pyran-3-carboxamido)ethyl benzoic acid YJ124.

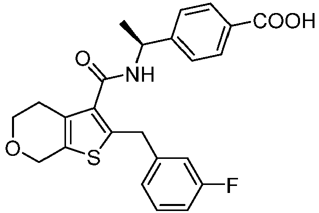

Compound YJ124 (82%, yield of final step) was finally prepared following the same chemical reaction procedure as the preparation of compound YJ114, except using p-chlorobenzaldehyde instead of p-fluorobenzaldehyde. ¹H NMR (500 MHz, DMSO) δ 12.83 (s, 1H), 8.74 (d, *J* = 8.0 Hz, 1H), 7.90 (d, *J* = 8.2 Hz, 2H), 7.46 (d, *J* = 8.1 Hz, 2H), 7.28 (d, *J* = 8.3 Hz, 2H), 7.16 (d, *J* = 8.3 Hz, 2H), 5.20 - 5.08 (m, 1H), 4.63 (s, 2H), 4.11 (m, *J* = 39.6, 15.5 Hz, 2H), 3.82 (m, *J* = 9.0, 5.4 Hz, 2H), 2.62 (d, *J* = 4.4 Hz, 2H), 1.41 (d, *J =* 7.0 Hz, 3H).

### Example 1-25. Preparation of (S)-4-(1-(2-(3-fluoro-4-methoxybenzyl)-4,7-dihydro-5H-thieno[2,3-c]pyran-3-carboxa mido)ethyl)benzoic acid (YJ125).

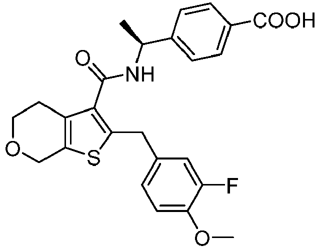

Compound YJ125 (79%, yield of final step) was finally prepared following the same chemical reaction procedure as the preparation of compound YJ114, except using 3-fluoro-4-methoxybenzaldehyde instead of p-fluorobenzaldehyde. ¹H NMR (500 MHz, DMSO) δ 12.81 (s, 1H), 8.75 (d, *J =* 7.9 Hz, 1H), 7.90 (d, *J =* 7.5 Hz, 2H), 7.47 (d, *J* = 7.6 Hz, 2H), 7.06 - 6.95 (m, 2H), 6.90 (d, *J* = 8.2 Hz, 1H), 5.20 - 5.10 (m, 1H), 4.64 (s, 2H), 4.06 (dd, *J* = 38.0, 15.5 Hz, 2H), 3.88 - 3.72 (m, 5H), 2.62 (d, *J* = 3.8 Hz, 2H), 1.42 (d, *J =* 6.8 Hz, 3H).

### Example 1-26. Preparation of (S)-4-(1-(2-(3-chlorobenzyl)-4,7-dihydro-5H-thieno[2,3-c]pyran-3-carboxamido)ethyl )benzoic acid (YJ126).

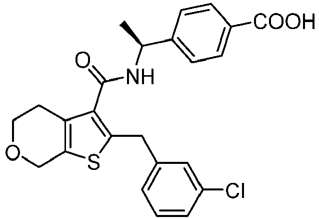

Compound YJ126 (81%, yield of final step) was finally prepared following the same chemical reaction procedure as the preparation of compound YJ114, except using m-chlorobenzaldehyde is used instead of p-fluorobenzaldehyde. ¹H NMR (500 MHz, DMSO) δ 12.26 (s, 1H), 8.78 (d, *J* = 7.9 Hz, 1H), 7.90 (d, *J* = 8.2 Hz, 2H), 7.47 (d, *J* = 8.2 Hz, 2H), 7.26 (m, *J* = 8.4 Hz, 3H), 7.13 (d, *J* = 6.5 Hz, 1H), 5.19 - 5.11 (m, 1H), 4.65 (s, 2H), 4.14 (m, *J* = 15.4 Hz, 2H), 3.83 (m, *J* = 5.3 Hz, 2H), 2.63 (d, *J* = 5.1 Hz, 2H), 1.42 (d, *J* = 7.0 Hz, 3H).

### Example 1-27. Preparation of (S)-4-(1-(2-(3,4-difluorobenzyl)-4,7-dihydro-5H-thieno[2,3-c]pyran-3-carboxamido)et hyl)benzoic acid (YJ127).

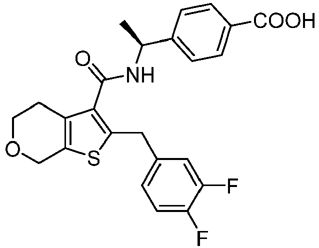

Compound YJ127 (81%, yield of final step) was finally prepared following the same chemical reaction procedure as the preparation of compound YJ114, except using 3,4-difluorobenzaldehyde instead of p-fluorobenzaldehyde. ¹H NMR (500 MHz, DMSO) δ 12.85 (s, 1H), 8.77 (d, *J* = 8.0 Hz, 1H), 7.89 (d, *J* = 8.2 Hz, 2H), 7.46 (d, *J* = 8.2 Hz, 2H), 7.34 - 7.27 (m, 1H), 7.26 - 7.17 (m, 1H), 7.00 (s, 1H), 5.19 - 5.11 (m, 1H), 4.65 (s, 2H), 4.12 (m, *J* = 31.8, 15.6 Hz, 2H), 3.83 (m, *J* = 8.2, 5.5 Hz, 2H), 2.63 (s, 2H), 1.42 (d, *J =* 7.0 Hz, 3H).

### Example 1-28. Preparation of (S)-4-(1-(2-(4-methoxybenzyl)-4,7-dihydro-5H-thieno[2,3-c]pyran-3-carboxamido)eth yl)benzoic acid (YJ128).

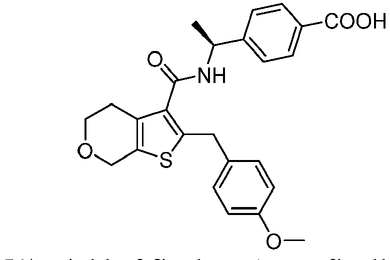

Compound YJ128 (85%, yield of final step) was finally prepared following the same chemical reaction procedure as the preparation of compound YJ114, except using 4-methoxybenzaldehyde instead of *p*-fluorobenzaldehyde. ¹H NMR (500 MHz, DMSO) δ 12.05 (s, 1H), 8.72 (d, *J* = 8.1 Hz, 1H), 7.90 (d, *J* = 8.2 Hz, 2H), 7.48 (d, *J =* 8.2 Hz, 2H), 7.04 (d, *J* = 8.5 Hz, 2H), 6.78 (d, *J* = 8.5 Hz, 2H), 5.20 - 5.11 (m, 1H), 4.62 (s, 2H), 4.04 (m, *J* = 48.1, 15.5 Hz, 2H), 3.81 (m, *J* = 11.0, 5.7 Hz, 2H), 3.70 (s, 3H), 2.64 - 2.58 (m, 2H), 1.43 (d, *J* = 12.6 Hz, 3H).

### Example 1-29. Preparation of ((2-(3-(trifluoromethyl)benzyl)-4,7-dihydro-5H-thieno[2,3-c]pyran-3-carboxamido)me thyl)benzoic acid (YJ129).

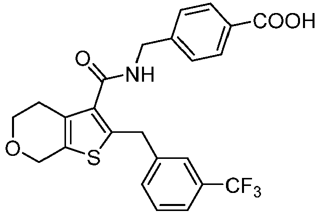

Compound YJ129 (85%, yield of final step) was finally prepared following the same chemical reaction procedure as the preparation of compound YJ120, except using 3-(trifluoromethyl)benzaldehyde instead of p-fluorobenzaldehyde. ¹H NMR (500 MHz, DMSO) δ 12.73 (s, 1H), 8.76 (t, *J* = 6.0 Hz, 1H), 7.81 (d, *J* = 8.0 Hz, 2H), 7.64 (s, 1H), 7.61 - 7.55 (m, 1H), 7.51 (d, *J* = 5.0 Hz, 2H), 7.23 (d, *J* = 8.0 Hz, 2H), 4.63 (s, 2H), 4.44 (d, *J* = 6.0 Hz, 2H), 4.29 (s, 2H), 3.82 (t, *J* = 5.5 Hz, 2H), 2.65 (t, *J* = 5.1 Hz, 2H).

### Example 1-30. Preparation of 4-((2-(4-fluorobenzyl)-4,7-dihydro-5H-thieno[2,3-c]pyran-3-carboxamido)methyl)cycl ohexane-1-carboxylic acid (racemates) (YJ130).

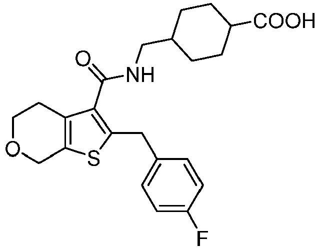

Compound YJ130 (90%, yield of final step) was finally prepared following the same chemical reaction procedure as the synthesis of compound YJ114, except using methyl 4-(aminomethyl)cyclohexane-1-carboxylate hydrochloride instead of methyl (S)-4-(1-aminoethyl)benzoate. ¹H NMR (500 MHz, DMSO) δ 12.02 (s, 1H), 8.16 (t, *J* = 5.4 Hz, 1H), 7.38 - 7.19 (m, 2H), 7.11 (t, *J =* 8.8 Hz, 2H), 4.62 (s, 2H), 4.14 (s, 2H), 3.82 (t, *J* = 5.3 Hz, 2H), 3.06 (t, *J* = 6.1 Hz, 2H), 3.06 (t, *J* = 6.1 Hz, 2H), 2.61 (s, 2H), 1.87 (d, *J* = 11.1 Hz, 2H), 1.73 (d, *J =* 11.4 Hz, 2H), 1.43 (d, *J* = 8.2 Hz, 2H), 1.24 (dd, *J* = 22.8, 12.6 Hz, 2H), 0.93 (dd, *J =* 23.5, 11.5 Hz, 2H).

### Example 1-31. Preparation of 4-(1-(2-(4-fluorobenzyl)-4,7-dihydro-5H-thieno[2,3-c]pyran-3-carboxamido)cyclopro pyl)benzoic acid (YJ131).

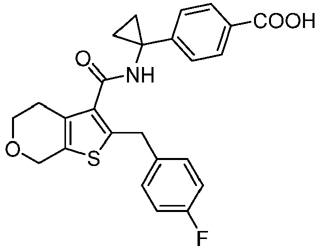

Compound YJ131 (80%, yield of final step) was finally prepared following the same chemical reaction procedure as the synthesis of compound YJ114, except using methyl 4-(1-aminocyclopropyl)benzoate instead of methyl
(S)-4-(1-aminoethyl)benzoate. ¹H NMR (500 MHz, DMSO) δ 12.81 (s, 1H), 8.96 (s, 1H), 7.82 (d, *J* = 8.3 Hz, 2H), 7.23 (m, *J =* 11.4, 5.6 Hz, 4H), 7.12 (t, *J* = 8.8 Hz, 2H), 4.65 (s, 2H), 4.18 (s, 2H), 3.84 (t, *J =* 5.4 Hz, 2H), 2.67 (s, 2H), 1.31 (d, *J =* 4.4 Hz, 2H), 1.24 (s, 2H).

### Example 2. The Evaluation of Inhibitory Effects of Compounds of The Present Invention on Calcium Flux of Human/Mouse Prostaglandin E2 Receptor Ep4 Subtype

CHO-K1-Gα16 cells at the logarithmic growth phase were transfected with EP4 overexpressing vector by Lipofectamine 2000 and then seeded in a 96-well plate (Falcon, CA) and cultured at 20000 cells per 100 µL culture medium overnight. On the second day, every single well were added with 100 µL calcium 5 detection reagents (Molecular Devices) and incubated in incubator at 37 °C, 5%CO₂ for 45 minutes.The compounds were diluted in HBSS detection buffer, then added to 96-well plates. After incubated for 20 min at room temperature away from light, certain concentration of prostaglandin E2 was used to activate calcium flux of cells for detection (Flexstation 3, MD, CA).

Calculate the IC₅₀ of the compounds of the invention in inhibiting the calcium flux of human PGE2 receptor EP4 subtype (hEP4) and mouse PGE2 receptor EP4 subtype (mEP4) (i.e.the concentration of drugs at which the intracellular Ca²⁺ flux is inhibited by half after overexpressing hEP4 and mEP4 rreceptors in CHO-K1-Gα16 cells). The IC₅₀ of the drugs were calculated using GraphPad Prism 5.

Table 2 shows the IC₅₀ of certain compounds of the invention on calcium flux assay of human/mouse prostaglandin E2 receptor EP4 subtype. As indicated in Table 2, most of the compounds prepared by the examples of the invention showed better inhibitory effect on calcium flux of human/mouse prostaglandin E2 receptor EP4 subtype.

**Table 2**

| | **hEP4 Ca²⁺ IC₅₀(nM)** | **mEP4 Ca²⁺ IC₅₀(nM)** |
|---|---|---|
| E7046 | A | B |
| YJ101 | A | B |
| YJ102** | A | A |
| YJ104** | A | B |
| YJ105** | B | - |
| YJ106 | A | A |
| YJ107** | A | B |
| YJ108** | A | A |
| YJ109** | A | A |
| YJ110 | A | A |
| YJ111 | A | A |
| YJ112 | A | B |
| YJ113 | A | B |
| YJ114 | A | A |
| YJ115 | A | A |
| YJ116 | A | A |
| YJ117 | B | - |
| YJ120 | A | A |
| YJ121 | A | A |
| YJ122 | A | - |
| YJ123 | A | - |
| YJ124 | A | - |
| YJ125 | A | - |
| YJ126 | A | - |
| YJ127 | A | - |
| YJ128 | A | - |
| YJ129 | A | - |
| YJ130 | A | B |
| YJ131 | A | A |

| | | |
|---|---|---|
| * A:<100 nM, B : 100-1000 nM, "-" means the activity not tested ** not part of the invention | | |

### Example 3. The Evaluation On Inhibitory Effects of Compounds of The Invention On Calcium Flux of Human Prostaglandin E2 Receptor Epl, Ep2, Ep3 Subtype

CHO-K1-Gα16 cells at the logarithmic growth phase were transfected with human prostaglandin E2 Receptor subtypes EP1, EP2 or EP3 overexpressing vector by Lipofectamine 2000, then seeded in a 96-well plate (Falcon, CA) at a density of 20000 cells per 100 µLculture medium for overnight culture. On the second day, every single well were added with 100 µL calcium 5 detection reagents (Molecular Devices) and incubated in incubator at 37 °C, 5%CO₂ for 45 minutes.The compounds were diluted with HBSS detection buffer, then were added to 96-well plates. After 20 min incubation at room temperature in the dark, prostaglandin E2 at certain concentration was used to activate calcium flux of cells for detection (Flexstation 3, MD, CA).

Calculate the IC₅₀ of the compounds of the invention in inhibiting the calcium flux of human EP1, EP2, and EP3 receptor (i.e.the concentration of drugs at which the intracellular Ca²⁺ flux being inhibited by half after overexpressing human EP1, EP2, and EP3 receptors in CHO-K1-Gα16 cells). The IC₅₀ of the drugs were calculated using GraphPad Prism 5.

Table 3 shows the IC₅₀ of certain compounds of the present invention on calcium flux of human prostaglandin E2 receptor EP1, EP2, and EP3 subtype. As indicated in Table 3, the inhibition IC₅₀ of most compounds prepared by the examples of the invention on human prostaglandin E2 receptor EP1, EP2, and EP3 subtype are more than 10 µM, possessing no antagonistic activity.

**Table 3**

| | **hEP1 Ca²⁺ IC₅₀(µM)** | **hEP2 Ca²⁺ IC₅₀(µM)** | **hEP3 Ca²⁺ IC₅₀(µM)** |
|---|---|---|---|
| E7046 | >10 | >10 | >10 |
| YJ101 | >10 | >10 | >10 |
| YJ104* | >10 | >10 | >10 |
| YJ106 | >10 | >10 | >10 |
| YJ108* | >10 | >10 | >10 |
| YJ109* | >10 | >10 | >10 |
| YJ110 | >10 | >10 | >10 |
| YJ112 | >10 | >10 | >10 |
| YJ113 | >10 | >10 | >10 |
| YJ114 | >10 | >10 | >10 |
| YJ115 | >10 | >10 | >10 |
| YJ116 | >10 | >10 | >10 |
| YJ120 | >10 | >10 | >10 |
| YJ121 | 1-10 | 1-10 | 1-10 |

| | | | |
|---|---|---|---|
| *not part of the invention | | | |

Both Example 2 and Example 3 indicated that compounds of the present invention have good specificity for EP receptors, they possess high inhibitory activity on EP4 receptor but have no antagonistic activity on EP1, EP2, and EP3 receptors.

### Example 4. Antagonistic Type Analysis for Compounds of The Present Invention On Human Prostaglandin E2 Receptor EP4

293 cells at the logarithmic growth phase were transfected with EP4 receptor overexpressing plasmid and Glosensor plasmid by Lipofectamine 2000, and incubated in incubator overnight at 37 °C with 5%CO₂. On the next day, cells were digested with trypsin and resuspended at the density of 15000 cells per 20 µL using HBSS detection buffer with 2% serumand GloSensor^{™} cAMP reagents (Promega). Cells were added at a concentration of 4%, after mixeture, seeded in 384-well plates at 20 µl/ well and incubated at room temperature away from light for 1.5 hours.The compounds were diluted with HBSS detection buffer, then were added to 384-well plates. After 20 min incubation at room temperature away from light, prostaglandin E2 at certain concentration was used to activate intracellular cAMP for detection (Flexstation 3, MD, CA).

Antagonists can be categorized into competitive antagonists and noncompetitive antagonists based on their different roles. Competitive antagonists of EP4 receptors can dose-dependently inhibit the cAMP upregulation level caused by prostaglandin E2 while the concentration of agonist prostaglandin E2 remains unchanged. When competitive antagonists reach a high concentration, they can totally inhibit the upregulation effect on cAMP caused by prostaglandin E2. At the same time, a sufficiently high concentration of prostaglandin E2 can abolish the effect of certain concentration of competitive antagonists and still make cAMP upregulated to maximum. The presence of competitive antagonists can make the concentration-response curve of the cAMP upregulation caused by prostaglandin E2 shift parallel right.

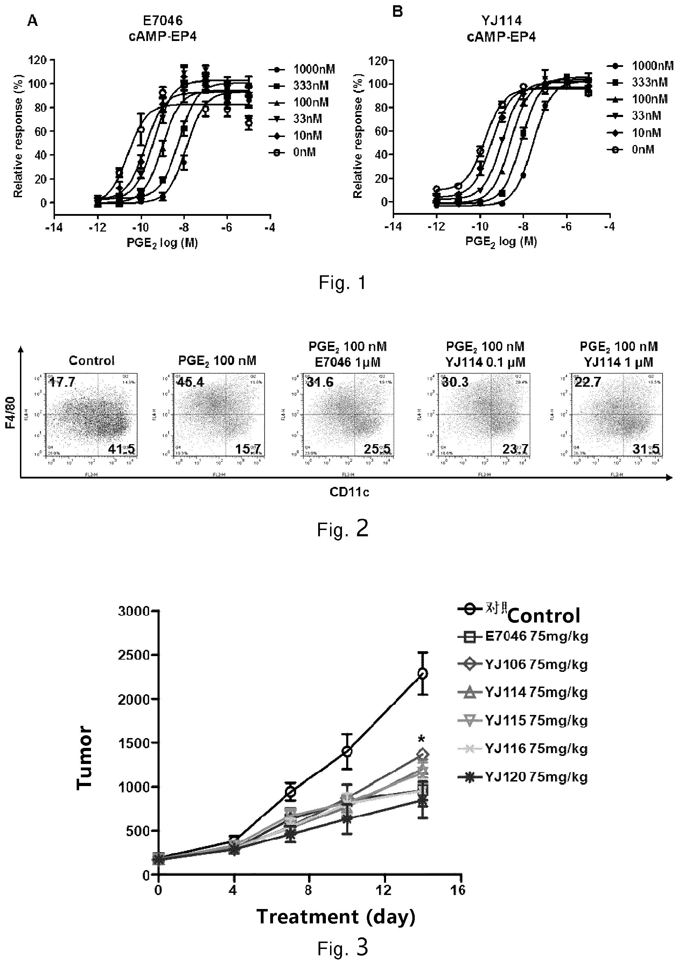

FIG. 1 shows concentration-response curves of certain compounds of the invention for detecting the extent of cAMP upregulation caused by human prostaglandin E2 receptor EP4 subtype. As shown in FIG. 1, as the concentrations of Compound YJ114 prepared according to the example of the invention increases, the concentration-response curve of the cAMP upregulation caused by prostaglandin receptor E2 shift parallel right while the maximum value remain unchanged, the same mechanism as the antagonist E7046. Hence, compounds of the present invention could serve as competitive antagonists of prostaglandin E2 receptor EP4 subtype.

### Example 5. Inhibitory Effect of Compounds of The Present Invention On Tumor Growth

Positive compound E7046 used in present invention is an EP4 antagonist developed by Eisai Company in Japan. Two clinical trials are currently in Phase I, respectively the assess of the safety and drug tolerance of E7046 in patients with advanced malignancy (NCT02540291) and evaluation of safety and tolerability of E7046 in combination with preoperative chemotherapy/radiotherapy for treating patients with advanced colon cancer (NCT03152370). The first clinical trial was terminated due to licensing agreement with Hangzhou Adlai Nortye pharmaceutical company. This compound possesses good antagonistic activity (IC₅₀=13 nM obtained by human EP4 receptor being stimulated with 8.5 nM PGE₂ and then inhibited with E7046; the affinity to human EP4 receptor was 17 nM and the affinity to mouse EP4 receptor was 200 nM). E7046 is able to suppress the growth of tumor by enhance the activity of T cells and myeloid-derived cells, and it has been proven that E7046 exhibits good inhibitory effects in mouse models of colon cancer, breast cancer, pancreatic cancer and sarcoma (Oncoimmunology, 2017, 6(8): e1338239.). E7046 differs very much from the compounds of the present invention, the structure of E7046 is shown as below:

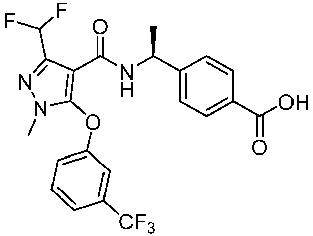

2×10⁶ mouse colon cancer cells CT26 were subcutaneously injected into right side of each mouse's back (female Balb/c mouse, 6-8 weeks). The length and width of subcutaneous tumors in the back of mice were measured using vernier calipers, and tumor volume (mm³)=(length(mm) x width(mm) x width(mm) x π)/6 was calculated. When the tumor volume reached 100-200 mm³, mice were separated into 7 groups randomly including negative control group, E7046 positive control group (75 mg/kg/day), Compound YJ106 treated group (75 mg/kg/day), Compound YJ114 treated group (75 mg/kg/day), Compound YJ115 treated group(75 mg/kg/day), Compound YJ116 treated group (75 mg/kg/day), and Compound YJ120 treated group (75 mg/kg/day), respectively. The volume of subcutaneous tumors on the back of mice was measured and counted twice a week. Continuous administrated for a period of 14 days and the changes of tumor volumes were recorded.

The results in FIG. 3 showing the inhibitory effect of compounds of the present invention on the growth of colon cancer in mice after two-week administration. Compared to the negative control group, groups of compound YJ106, compound YJ114, compound YJ115, compound YJ116 and compound YJ120 were able to suppress the growth of Subcutaneous tumor-bearing colon cancer with a dosage of 75 mg/kg/day. The inhibitory effect of the compounds of the invention is comparable with that of E7046 at the same dosage. After statistical analysis and verification, the P values between the tumor volume of treated groups (compound YJ106, compound YJ114, compound YJ115, compound YJ116 and compound YJ120) and that of negative control group are < 0.05 (P value is a parameter used in statistical analysis for determining the results of hypothesis test results, the smaller the P value is, the more significant the result is), that is to say there are significant differences. These results of statistical analysis are the same as E7046 positive control group .

### Example 6. Inhibitory Effects of Compound YJ114 of The Present Invention On The Growth of Various Cancer Types

Mouse colon cancer cells MC38, pancreatic cancer cells Pan02, breast cancer cells 4T1, prostate cancer cells RM-1 and colon cancer cells CT26 were cultivated to required amount. Then, cells at a cell density of 5×10⁵-2×10⁶ were subcutaneously injected into right side of the back of selected female C57BL/6 mice aging 6-8 weeks (MC38, Pan02), male C57BL/6 mice aging 6-8 weeks (RM-1), female Balb/c mice aging 6-8 weeks (4T1, CT26) and female Balb/c nude mice aging 6-8 weeks (CT26). The length and width of subcutaneous tumors in the backs of mice were measured using vernier calipers, and tumor volume(mm³)= (length(mm) x width(mm) x width(mm) x π)/6 was calculated. When the tumor volume reached 100-200 mm³, the mice were divided into two groupsrandomly, including negative control group and Compound YJ114 treated group (75 mg/kg/day). The volume of subcutaneous tumors on the back of mice was measured and counted 2-4 times a week. Continuous administrated for 12 -22 days and the changes of tumor volumes were recorded.

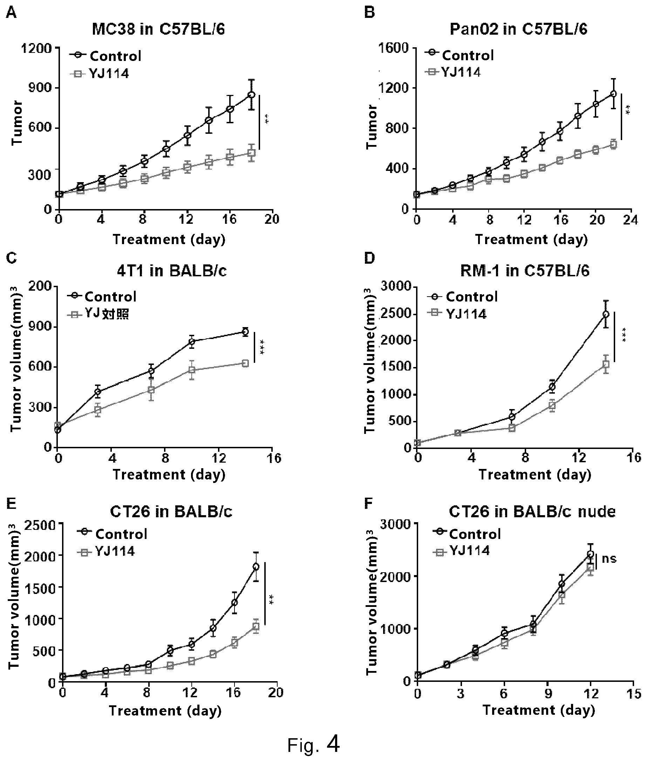

The results in FIG. 4 showing the inhibitory effects of Compound YJ114 on the growth of various mouse tumors after 12 to 22 - day administration. Compared to the negative control group, Compound YJ114 with a dosage of 75mg/kg/day can efficiently inhibit the growth of mouse colon cancer cells MC38, pancreatic cancer cells Pan02, breast cancer cells 4T1, prostate cancer cells RM-1 and colon cancer cells CT26 in subcutaneous tumor-bearing mice. After statistical analysis and verification, all of the P values between the tumor volume of each Compound YJ114 treated group and that of negative control group are < 0.05, that is to say there are significant differences. However, in the CT26 cell subcutaneous tumor-bearing model of Balb/c nude mice with immune system deficiency, Compound YJ114 of the invention at a dosage of 75 mg / kg / day can not inhibit the tumor growth, the P value between the tumor volume of treated group and that of negative control group is > 0.05 and there is no significant difference.

These results above showed that Compound YJ114 can significantly suppress tumor growth in mouse subcutaneous tumor-bearing model with an intact immune system, while Compound YJ114 is unable to suppress tumor growth in Balb/c nude mice with immune deficiency. It is indicated that Compound YJ114 of the invention relies on intact immune system to inhibit tumor growth *in vivo.*

### Example 7. Cytotoxicity Evaluation of Compounds of The Present Invention

Mouse osteosarcoma cells MOSJ, pancreatic cancer cell PANC02, breast cancer cells EMT-6, lung cancer cells LLC, intestinal cancer cells CT26, and human renal epithelial cell line 293 transfected with adenovirus E1A gene (Tool Cell 293) were used for the cytotoxicity evaluation of E7046 and the compounds of the invention. Cells with a density of 2000-8000 cells per 100 µL medium were seeded in 96-well plates (Falcon, CA) and incubated for 24 hours, then treated with drugs having different concentration gradient (at least 4 replicates per concentration) and incubated at 37 °C in 5% CO₂ incubator for 72 hours. Then cell viability was detected by CCK8 method. That is to say, add 10 µL CCK8 to each well and incubate for 0.33-2 h, and the OD values are read by microplate reader at 450 nm. The inhibition rate on cell survival of compounds with each concentration were calculated.

Inhibition rate on cell survival = (OD value of groups with each concentration/ OD value of control group) x100% ± standard deviation.

Compound's IC₅₀ for cell growth inhibition (the concentration at which 50% of the test cells growth are inhibited is IC₅₀) were further calculated. The IC₅₀ of drugs were calculated using GraphPad Prism 5.0.

Table 4 showed the IC₅₀ values of certain compounds prepared by the examples of the invention for inhibiting the growth of mouse cancer cells and tool cells 293.

As shown in Table 4, IC₅₀ of most of compounds of the invention on mouse cancer cells and tool cells 293 are > 100 µM. The above results showed that compounds of the invention have no killing effect on cancer cells themselves. Therefore, it is speculated that the *in vivo* inhibitory effects of compounds on tumor growth were obtained via immune system.

**Table 4**

| | **Cytotoxicity-MTS IC₅₀(nM)** | | | | | |
|---|---|---|---|---|---|---|
| | MOSJ | PANC02 | EMT-6 | LLC | CT-26 | 293 |
| E7046 | > 10⁵ | > 10⁵ | > 10⁵ | >10⁵ | > 10⁵ | > 10⁵ |
| YJ101 | > 10⁵ | > 10⁵ | > 10⁵ | >10⁵ | > 10⁵ | > 10⁵ |
| YJ104* | > 10⁵ | > 10⁵ | > 10⁵ | >10⁵ | > 10⁵ | > 10⁵ |
| YJ106 | > 10⁵ | > 10⁵ | > 10⁵ | >10⁵ | > 10⁵ | > 10⁵ |
| YJ108* | > 10⁵ | > 10⁵ | > 10⁵ | >10⁵ | > 10⁵ | > 10⁵ |
| YJ 109 * | > 10⁵ | > 10⁵ | > 10⁵ | >10⁵ | > 10⁵ | > 10⁵ |
| YJ110 | > 10⁵ | > 10⁵ | > 10⁵ | >10⁵ | > 10⁵ | > 10⁵ |
| YJ112 | > 10⁵ | > 10⁵ | > 10⁵ | >10⁵ | > 10⁵ | > 10⁵ |
| YJ113 | > 10⁵ | > 10⁵ | > 10⁵ | >10⁵ | > 10⁵ | > 10⁵ |
| YJ114 | > 10⁵ | > 10⁵ | > 10⁵ | >10⁵ | > 10⁵ | > 10⁵ |
| YJ115 | > 10⁵ | > 10⁵ | > 10⁵ | >10⁵ | > 10⁵ | > 10⁵ |
| YJ116 | > 10⁵ | > 10⁵ | > 10⁵ | >10⁵ | > 10⁵ | > 10⁵ |
| YJ 120 | > 10⁵ | > 10⁵ | > 10⁵ | >10⁵ | > 10⁵ | > 10⁵ |
| YJ121 | 10⁴-10⁵ | 10⁴-10⁵ | 10⁴-10⁵ | 10⁴-10⁵ | 10⁴-10⁵ | 10⁴-10⁵ |
| gemcitabine | - | 72.6±0.3 | - | 5.3±0.2 | 16.0±0.1 | 5.1±0.1 |

| | | | | | | |
|---|---|---|---|---|---|---|
| * not part of the invention | | | | | | |

### Example 8 The Assessment of Compounds of Present Invention on Mouse Myeloid Cell Differentiation

6-8 weeks BALB/c mice were sacrificed by cervical dislocation and immersed in 75% ethanol for 5 minutes. The bilateral femur and tibia were isolated, and soaked in sterile PBS after fully peeling off the attached muscles. Each end of bone was cut off under sterile condition. The bone marrow cavity was repeatedly flushed with RPMI 1640 medium (Gibco) drawn by 1 mL syringe until the color of the bones turned white. Collected myeloid cells suspension was filtrated with 40 µm filter to remove tissue fragments, and the cells were fully dispersed. Centrifuge at 1000 rpm for 5 minutes, the supernatant was discarded, erythrocytes were fully lysed by adding red blood cells lysis buffer and then centrifuged. The cells was re-suspended to a density of 1×10⁶ cells/mL with RPMI-1640 medium, and seeded into 6-well plate (corning) at 4 mL/well, added GM-CSF (10 ng/mL) and IL-4 (5 ng/mL) (Sigma, MO), and added prostaglandin E2 and test compounds with different treat conditions at the same time. After cultured for 2 days, replace with the culture medium with the same conditions as above. On the 4th and 6th day, half-volume exchange was performed. When cultured to the 8th day, suspension cells and adherent cells were collected and incubated with F4/80 (macrophage surface marker) and CD11c (DC surface marker) for 30 minutes, after that analysis was performed with flow cytometry(FACS Calibur, BD Biosciences).

FIG. 2 shows the evaluation of the effect of compound YJ114 of present invention on the differentiation of mouse bone marrow cells. All of DC (Dendritic cells), MDSC and tumor-associated macrophages are derived from bone marrow mononuclear cells. While in clinical patient tumors, the level of prostaglandin E2 (PGE₂) often positively correlates with the accumulation of immunosuppressive cells MDSC and M2 tumor-associated macrophage. Hence, we further tested whether compound YJ114 of the invention affects the differentiation of myeloid cells to antigen presenting cells. As indicated in FIG. 2, the addition of 100 nM PGE2 can significantly suppress the generation of GM-CSF/IL-4-induced DC. However, the addition of 100 nM PGE₂ as well as Compound YJ114 of the invention at the same time can abolish the inhibitory effect of PGE2 against the generation of DC and shows a good concentration-dependence.Compared with the same concentration of the antagonist E7046, compound YJ114 of the invention showed a better efficacy on regulation of the differentiation of mouse bone marrow cells.

## Claims

1. A compound of formula (I): 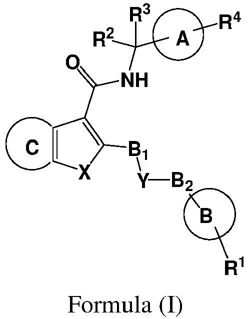 or pharmaceutically acceptable salts or hydrates thereof, wherein:
Ⓐ and Ⓑ are each independently selected from the group consisting of C3-C6 carbon ring, benzene ring, and 5- or 6-membered heteroaromatic ring having one or more O, N, S atoms, wherein Ⓐ and Ⓑ can be optionally substituted by 1 to 3 R⁵ substituents;
Ⓒis a substituted or unsubstituted ring selected from the group consisting of a 4-7 membered saturated heterocyclic ring, wherein the heterocyclic ring comprises one or more heteroatoms selected from O;
Xis -S-;
Y is absent, or a group selected from the group consisting of -CH₂-, -O-, -S-, -SO-, -SO₂-, and -N(R⁸)-;
B₁ and B₂ are groups each independently selected from the group consisting of absent, C1-C6 alkylene, C2-C6 alkenylene, C2-C6 alkynylene; and B₁, B₂ and Y are not absent at the same time;
R¹ is one or more group selected from the group consisting of H, C1-C6 alkyl, halogen, nitro, -N(R⁹)(R¹⁰), -OH, -CN, C1-C6 haloalkyl, C1-C6 alkoxy, and C1-C6 haloalkoxy;
R² and R³ are each independently selected from the group consisting of H, halogen, C1-C6 alkyl, C1-C6 haloalkyl, and C3-C6 cycloalkyl; or R², R³ together with the carbon atom to which they are attached form 3 to 6 membered ring, which is carbon ring or heterocyclic ring including 1 to 3 heteroatoms selected from O, S or N(R¹¹);
R⁴ is -COOR¹²,
R⁵ is selected from: H, halogen, C1-C6 alkyl, C1-C6 haloalkyl, C3-C6 cycloalkyl, and C1-C6 alkoxy;
R¹² is selected from the group consisting of H and C1-C6 alkyl;
R⁸, R⁹, R¹⁰ and R¹¹ are each independently selected from: H, C1-C6 alkyl, C1-C6 haloalkyl, C3-C6 cycloalkyl, C3-C6 halocycloalkyl, C6-C10 aryl, 5- or 6- membered heteroaryl, 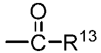 , and 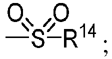
unless otherwise specified, one or more hydrogen atoms of the substituted groups are substituted with substituent(s) selected from the group consisting of F, Cl, Br, I, hydroxyl, methyl, ethyl, isopropyl, methoxy, ethoxy, trifluoromethyl, difluoromethoxy, trifluoromethoxy, nitro, -CN, oxo (= O);
R¹³ and R¹⁴ are each independently selected from: H, C1-C6 alkyl, C6-C10 aryl, C1-C6 alkylene, and -C6-C10 aryl.

2. The compound of claim 1, or pharmaceutically acceptable salts or hydrates thereof, wherein 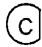 is a substituted or unsubstituted 6-membered oxa-heterocycle.

3. The compound of claim 1, or pharmaceutically acceptable salts or hydrates thereof, wherein 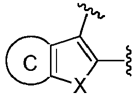 has a structure as shown in following formula: 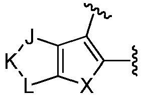
wherein one of J, K, and L is -O-; and the rest of J, K, and L are selected from the group consisting of -CH₂-, -CH(CH₃)-, -CH(CH₂CH₃)-, -C(CH₃)₂-, 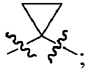 or 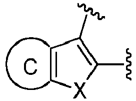 has a structure as shown in following formula: 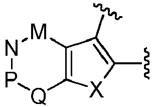
wherein one of M, N, P, and Q is -O-; and the rest of M, N, P, and Q are each independently selected from the group consisting of -CH₂-, -CH(CH₃)-, -CH(CH₂CH₃)-, -C(CH₃)₂-, and 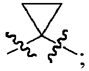
or 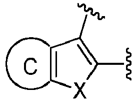 has a structure as shown in following formula: 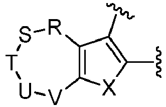
wherein one of R, S, T, U, and V is -O-; and the rest of R, S, T, U, and V are each independently selected from the group consisting of -CH₂-, -CH(CH₃)-, -CH(CH₂CH₃)-, -C(CH₃)₂-, and

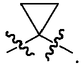

4. The compound of claim 1, or pharmaceutically acceptable salts or hydrates thereof, wherein 1 or 2 hydrogen atoms on the ring Ⓒ is substituted by a substituent selected from the group consisting of methyl, ethyl, and isopropyl.

5. The compound of claim 1, or pharmaceutically acceptable salts or hydrates thereof, wherein 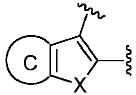 has a structure as shown in following formula: 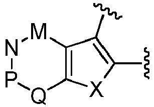
wherein one of M, N, P, and Q is -O-; and the rest of M, N, P, and Q are each independently selected from the group consisting of -CH₂-, -CH(CH₃)-, -CH(CH₂CH₃)-, -C(CH₃)₂-, and 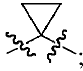 and
X is -S-.

6. The compound of claim 1, or pharmaceutically acceptable salts or hydrates thereof, wherein Ⓐ and Ⓑ are each independently selected form the group consisting of benzene ring, and 5- or 6-membered heteroaromatic ring comprising one or more O, N, S atoms, wherein Ⓐ and Ⓑ can optionally be substituted by 1 to 3 R⁵ substituents.

7. The compound of claim 1, or pharmaceutically acceptable salts or hydrates thereof, wherein Ⓐand Ⓑ are are each independently selected from the group consisting of benzene ring, and 5- or 6-membered heteroaromatic ring, wherein 5- or 6-membered heteroaromatic ring is selected form the group consisting of pyridine, pyrimidine, thiazole, isothiazole, furan, thiophene, and pyrrole; wherein Ⓐ and Ⓑ can optionally be substituted by 1 to 3 R⁵ substituents.

8. The compound of claim 1, or pharmaceutically acceptable salts or hydrates thereof, wherein R⁴ is selected from any one of the following groups: -COOH, -COOCH₃, -COOCH₂CH₃, -COOCH₂CH₂CH₃, -COOCH(CH₃)₂.

9. The compound of claim 1, or pharmaceutically acceptable salts, or hydrates thereof, wherein,
B₁ and B₂ are each independently selected from the group consisting of -(CH₂)ₙ-, wherein n=0, 1, 2, 3 or 4, -CH=CH-, -CH=CH-CH₂-, -CH₂-CH=CH-, -CH=CH-CH₂-CH₂-, -CH₂-CH=CH-CH₂-, -CH₂-CH₂-CH=CH-; -C≡C-, -C≡C-CH₂-, -CH₂-C≡C-, -C≡ C-CH₂-CH₂-, -CH₂-C≡C-CH₂-, -CH₂-CH₂-C≡C-, provided that B₁ and B₂ are not -(CH₂)ₙ- at the same time, wherein n=0.

10. The compound of claim 1, or pharmaceutically acceptable salts or hydrates thereof, wherein B₂ is -(CH₂)ₙ-, wherein n=0, and B₁ is selected from the group consisting of -(CH₂)ₙ-; wherein n=1, 2, 3 or 4, -CH=CH-, -CH=CH-CH₂-, -CH₂-CH=CH-, -CH=CH-CH₂-CH₂-, -CH₂-CH=CH-CH₂-, -CH₂-CH₂-CH=CH-; -C=C-, -C=; and Y is absent.

11. The compound of claim 1, or pharmaceutically acceptable salts or hydrates thereof, wherein the compound is selected from the group consisting of:
| | |
|---|---|
| 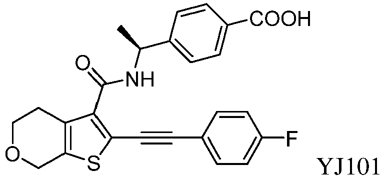 | |
| 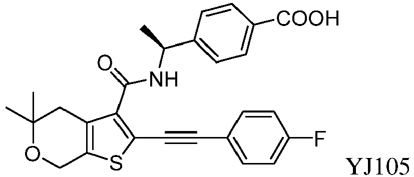 | 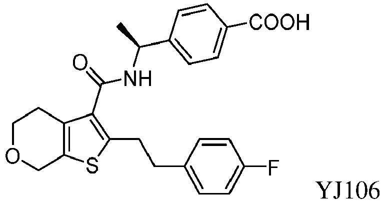 |
| | 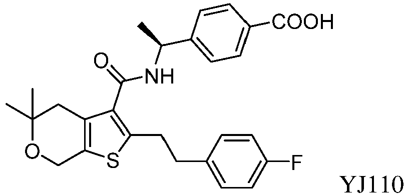 |
| 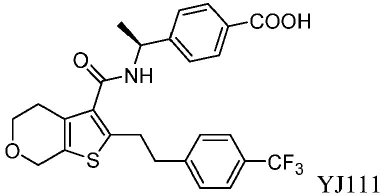 | 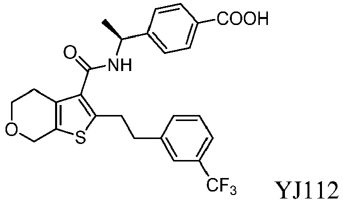 |
| 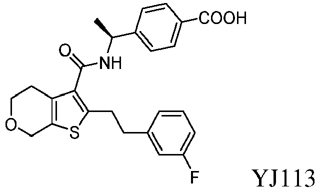 | 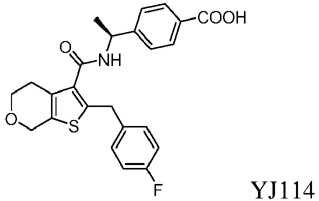 |
| 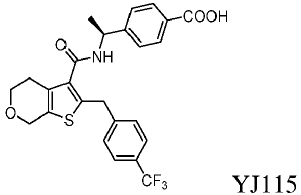 | 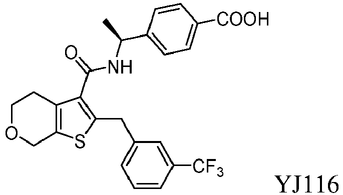 |
| 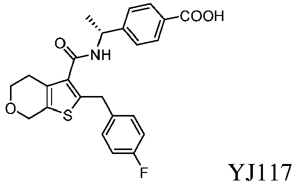 | 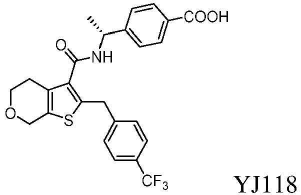 |
| 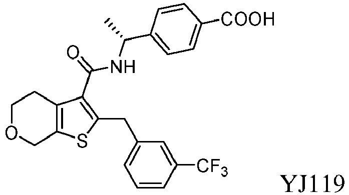 | 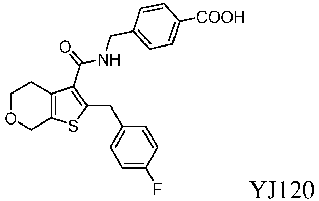 |
| 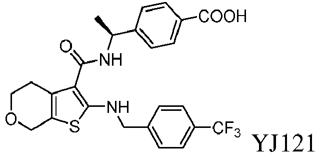 | 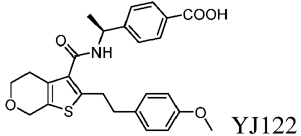 |
| 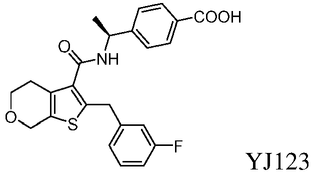 | 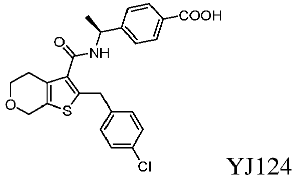 |
| 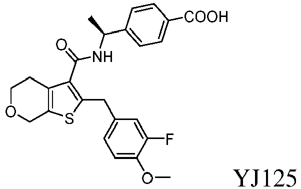 | 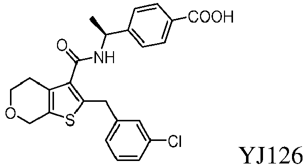 |
| 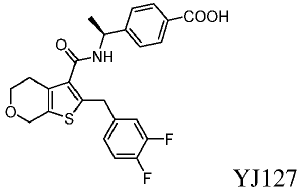 | 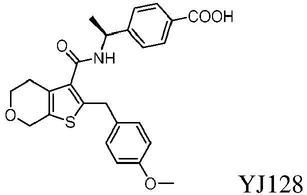 |
| 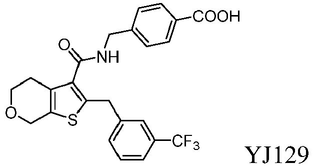 | 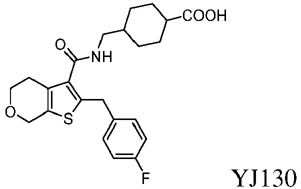 |
| 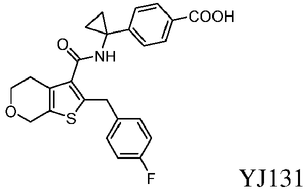 | |

12. A pharmaceutical composition, wherein the pharmaceutical composition comprises: a therapeutically effective amount of the compound of formula (I) according to claim 1, or pharmaceutically acceptable salts or hydrates thereof; and pharmaceutically acceptable carriers.

13. The compound of formula (I) according to claim 1, or pharmaceutically acceptable salts or hydrates thereof for use in:
(i) antagonizing prostaglandin receptor -4 (EP4);
(ii) inhibiting prostaglandin PGE 2;
(iii) enhancing *in vivo* immunocompetence of human or mammals, or preventing and /or treating prostaglandin PGE2-mediated diseases;
(iv) reducing side effects of non-steroidal anti-inflammatory drugs and cyclooxygenase-2 inhibitors against cardiovascular systems and gastrointestinal systems.

14. The compound for the use of claim 13, wherein the prostaglandin PGE2-mediated diseases are selected from the group consisted of autoimmune diseases, allergy, inflammation, bone diseases, acute or chronic pain, and tumor.

15. The compound for the use of claim 14, wherein the tumor is selected from the group consisting of liver cancer, lung cancer, prostate cancer, skin cancer, colon cancer, pancreatic cancer, breast cancer, leukemia, lymphoma, ovarian cancer, stomach cancer, bladder cancer, kidney cancer, oral cancer, melanoma, esophageal cancer, lymphoma, and cervical cancer.

## Patentansprüche

1. Verbindung von Formel (I): 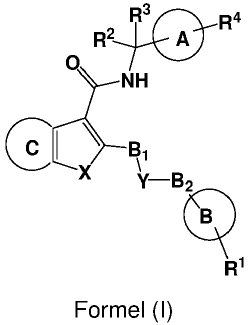 oder pharmazeutisch annehmbare Salze oder Hydrate davon, wobei:
Ⓐ und Ⓑ jeweils unabhängig ausgewählt sind aus der Gruppe, bestehend aus einem C3-C6-Kohlenstoffring, einem Benzolring und einem 5- oder 6-gliedrigen heteroaromatischen Ring, der ein oder mehrere O-, N- und S-Atome aufweist, wobei Ⓐ und Ⓑ optional durch 1 bis 3 R⁵-Substituenten substituiert sein können;
Ⓒ ein substituierter oder unsubstituierter Ring ist, der ausgewählt ist aus der Gruppe, bestehend aus einem 4-7-gliedrigen gesättigten heterocyclischen Ring, wobei der heterocyclische Ring ein oder mehrere Heteroatome umfasst, die ausgewählt sind aus O;
X S ist;
Y abwesend oder eine Gruppe ist, ausgewählt aus der Gruppe bestehend aus CH₂-, -O-, - S-, -SO-, -SO₂- und -N(R⁸)-;
B, and B₂ Gruppen sind, die jeweils unabhängig ausgewählt sind aus der Gruppe, bestehend aus abwesend, C1-C6-Alkylen, C2-C6-Alkenylen, C2-C6-Alkinylen; und B₁, B₂ und Y nicht gleichzeitig abwesend sind;
R¹ eine oder mehrere Gruppen ist, ausgewählt aus der Gruppe bestehend aus H, C1-C6-Alkyl, Halogen, Nitro, -N(R⁹)(R¹⁰), -OH, -CN, C₁-C₆-Halogenalkyl , C1-C6-Alkoxy und C1-C6-Halogenalkoxy;
R² und R³ jeweils unabhängig ausgewählt sind aus der Gruppe, bestehend aus H, Halogen, C1-C6-Alkyl, C1-C6-Halogenalkyl und C3-C6-Cycloalkyl; oder R², R³ zusammen mit dem Kohlenstoffatom, an das sie angelagert sind, einen 3- bis 6-gliedrigen Ring bilden, der ein Kohlenstoffring oder ein heterocyclischer Ring ist, der 1 bis 3 Heteroatome, ausgewählt aus O, S oder N(R¹¹), beinhaltet;
R⁴-COOR¹² ist;
R⁵ ausgewählt ist aus: H, Halogen, C1-C6-Alkyl, C1-C6-Halogenalkyl, C3-C6-Cycloalkyl und C1-C6-Alkoxy;
wobei R¹² ausgewählt ist aus der Gruppe bestehend aus H und C1-C6-Alkyl,
R⁸, R⁹, R¹⁰ und R¹¹ jeweils unabhängig ausgewählt sind aus: H, C1-C6-Alkyl, C1-C6-Halogenalkyl, C3-C6-Cycloalkyl, C3-C6-Halogencycloalkyl, C6-C10-Aryl, 5- oder 6-gliedrigem Heteroaryl, 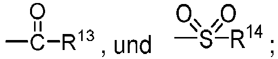
sofern nicht anders angegeben, ein oder mehrere Wasserstoffatome der substituierten Gruppen mit einem oder mehreren Substituenten substituiert sind, ausgewählt aus der Gruppe, bestehend aus F, Cl, Br, I, Hydroxyl, Methyl, Ethyl, Isopropyl, Methoxy, Ethoxy, Trifluormethyl, Difluormethoxy, Trifluormethoxy, Nitro, -CN, Oxo (= O);
R¹³ und R¹⁴ jeweils unabhängig ausgewählt sind aus: H, C1-C6-Alkyl, C6-C10-Aryl, C1-C6-Alkylen und -C6-C10-Aryl.

2. Verbindung nach Anspruch 1 oder pharmazeutisch annehmbare Salze oder Hydrate davon, wobei Ⓒ ein substituierter oder unsubstituierter 6-gliedriger Oxa-Heterocyclus ist.

3. Verbindung nach Anspruch 1 oder pharmazeutisch annehmbare Salze oder Hydrate davon,
wobei 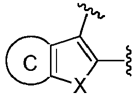 eine Struktur wie gezeigt in der folgenden Formel aufweist: 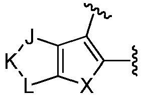
wobei eines von J, K und L -O- ist; und der Rest von J, K und L ausgewählt ist aus der Gruppe bestehend aus -CH₂-, -CH(CH₃)-, -CH(CH₂CH₃)-, -C(CH₃)₂-, 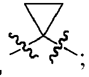
oder 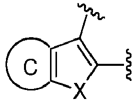 eine Struktur wie gezeigt in der folgenden Formel aufweist: 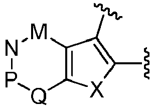
wobei eines von M, N, P und Q -O- ist; und der Rest von M, N, P und Q jeweils unabhängig ausgewählt ist aus der Gruppe, bestehend aus -CH₂-, -CH(CH₃)-, -CH(CH₂CH₃)-, -C(CH₃)2- und 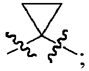
oder 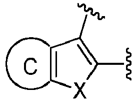 eine Struktur wie gezeigt in der folgenden Formel aufweist: 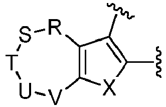
wobei eines von R, S, T, U und V -O- ist; und der Rest von R, S, T, U und V jeweils unabhängig ausgewählt ist aus der Gruppe, bestehend aus -CH2-, -CH(CH3)-, -CH(CH2CH3)-, - C(CH₃)2- und

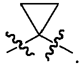

4. Verbindung nach Anspruch 1 oder pharmazeutisch annehmbare Salze oder Hydrate davon, wobei 1 oder 2 Wasserstoffatome an dem Ring Ⓒ durch einen Substituenten substituiert sind, ausgewählt aus der Gruppe, bestehend aus Methyl, Ethyl und Isopropyl.

5. Verbindung nach Anspruch 1 oder pharmazeutisch annehmbare Salze oder Hydrate davon,
wobei 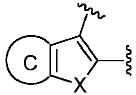 eine Struktur wie gezeigt in der folgenden Formel aufweist: 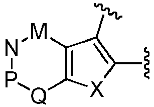
wobei eines von M, N, P und Q -O- ist; und der Rest von M, N, P und Q jeweils unabhängig ausgewählt ist aus der Gruppe, bestehend aus -CH2-, -CH(CH3)-, -CH(CH2CH3)-, -C(CH3)2- und 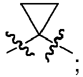 und
X -S- ist.

6. Verbindung nach Anspruch 1 oder pharmazeutisch annehmbare Salze oder Hydrate davon, wobei Ⓐ und Ⓑ jeweils unabhängig ausgewählt sind aus der Gruppe, bestehend aus einem Benzolring und einem 5- oder 6-gliedrigen heteroaromatischen Ring, umfassend ein oder mehrere O-, N-, S-Atome, wobei Ⓐ und Ⓑ optional durch 1 bis 3 R⁵-Substituenten substituiert sein können.

7. Verbindung nach Anspruch 1 oder pharmazeutisch annehmbare Salze oder Hydrate davon, wobei Ⓐ und Ⓑ jeweils unabhängig ausgewählt sind aus der Gruppe, bestehend aus einem Benzolring und einem 5- oder 6-gliedrigen heteroaromatischen Ring, wobei der 5oder 6-gliedrige heteroaromatische Ring ausgewählt ist aus der Gruppe, bestehend aus Pyridin, Pyrimidin, Thiazol, Isothiazol, Furan, Thiophen und Pyrrol; wobei Ⓐ und ^{®} optional durch 1 bis 3 R⁵-Substituenten substituiert sein können.

8. Verbindung nach Anspruch 1 oder pharmazeutisch annehmbare Salze oder Hydrate davon, wobei R⁴ ausgewählt ist aus einer der folgenden Gruppen: -COOH, -COOCH3, - COOCH2CH3, -COOCH2CH2CH3, -COOCH(CH₃)2.

9. Verbindung nach Anspruch 1 oder pharmazeutisch annehmbare Salze oder Hydrate davon, wobei
B1 und B2 jeweils unabhängig ausgewählt sind aus der Gruppe bestehend aus - (CH2)ₙ-, wobei n=0, 1, 2, 3 oder 4, -CH=CH-, -CH=CH-CH₂-, -CH₂-CH=CH-, -CH=CH-CH₂-CH₂-, -CH₂-CH=CH-CH₂-, -CH₂-CH₂-CH=CH-; -C≡C-, -C≡C-CH₂-, -CH₂-C≡C-, -C≡C-CH2-CH2-, -CH2-C=C-CH2-, -CH2-CH2-C=C-, mit der Maßgabe, dass B₁ and B₂ nicht gleichzeitig -(CH2)n- sind, wobei n=0.

10. Verbindung nach Anspruch 1 oder pharmazeutisch annehmbare Salze oder Hydrate davon, wobei B2 -(CH2)n- ist, wobei n=0, und B1 ausgewählt ist aus der Gruppe, bestehend aus - (CH2)ₙ-; wobei n=1, 2, 3 oder 4, -CH=CH-, -CH=CH-CH₂-, -CH₂-CH=CH-, -CH=CH-CH₂-CH₂-, -CH₂-CH=CH-CH₂-, -CH₂-CH₂-CH=CH-; -C=C-, -C=; und Y abwesend ist.

11. Verbindung nach Anspruch 1 oder pharmazeutisch annehmbare Salze oder Hydrate davon, wobei die Verbindung ausgewählt ist aus der Gruppe bestehend aus:

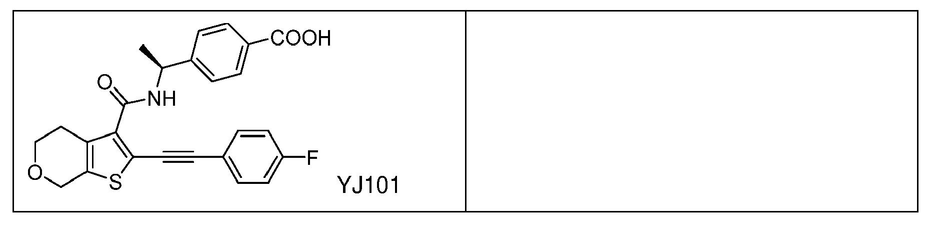

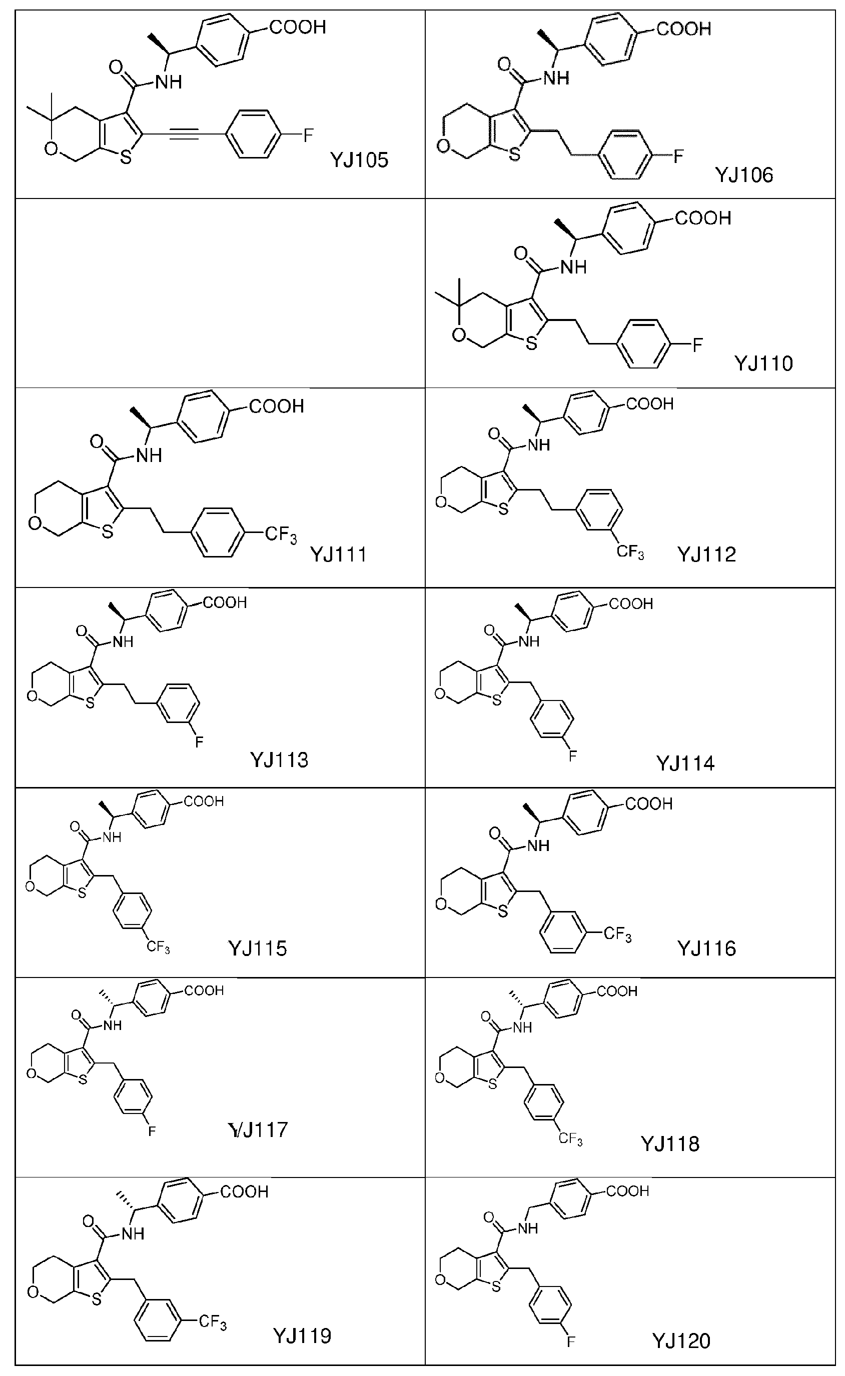

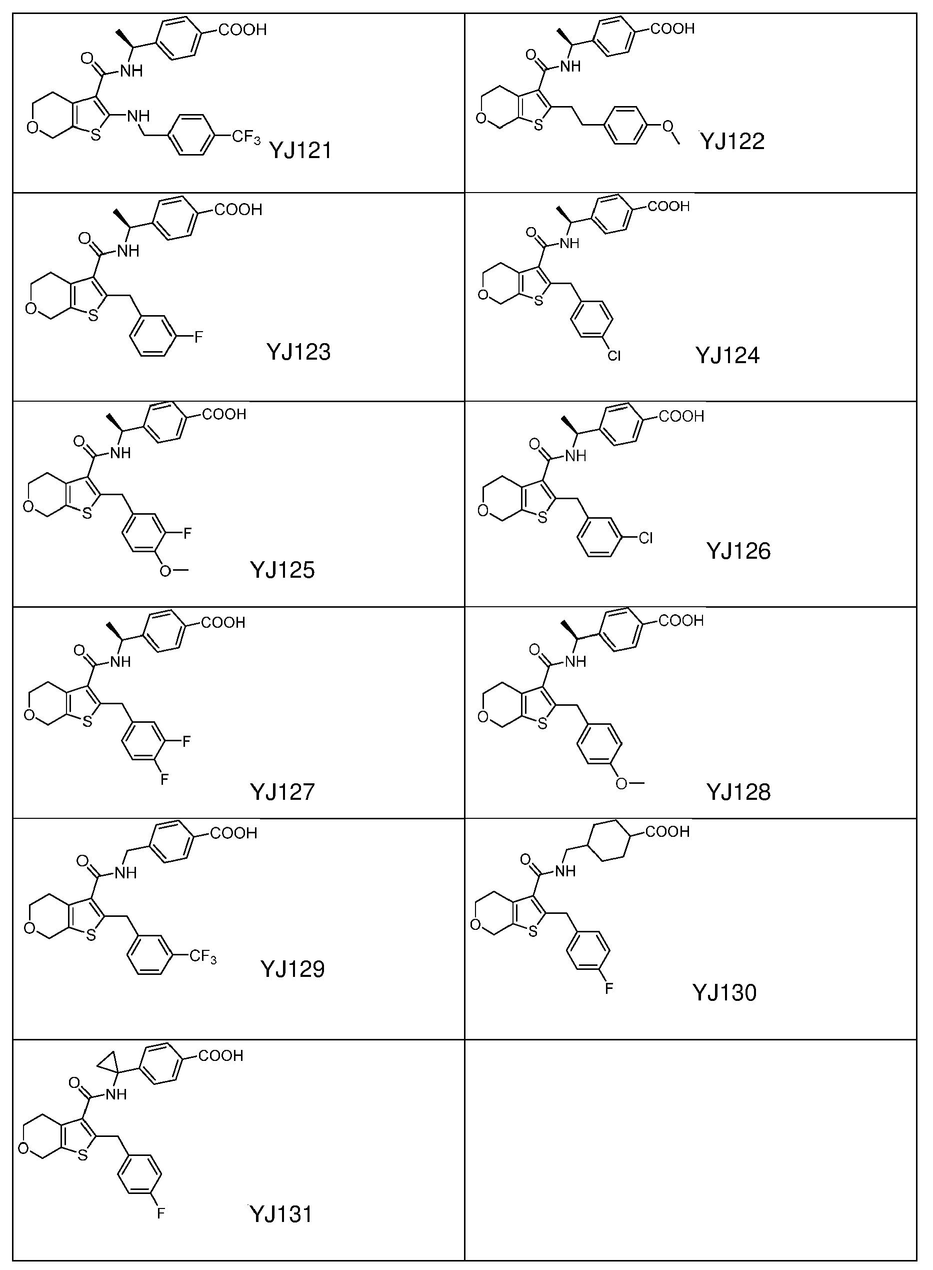

12. Pharmazeutische Zusammensetzung, wobei die pharmazeutische Zusammensetzung Folgendes umfasst: eine therapeutisch wirksame Menge der Verbindung der Formel (I) nach Anspruch 1 oder pharmazeutisch annehmbare Salze oder Hydrate davon; und pharmazeutisch annehmbare Träger.

13. Verbindung von Formel (I) nach Anspruch 1 oder pharmazeutisch annehmbare Salze oder Hydrate davon zur Verwendung bei:
(i) Antagonisieren von Prostaglandinrezeptor -4 (EP4);
(ii) Hemmen von Prostaglandin PGE 2;
(iii) Verbessern von in-vivo-Immunkompetenz von Menschen oder Säugetieren oder Vorbeugung und/oder Behandlung von Prostaglandin-PGE2-vermittelten Krankheiten;
(iv) Verringern der Nebenwirkungen von nichtsteroidalen Entzündungshemmern und Cyclooxygenase-2-Hemmern auf das Herz-Kreislauf-System und den Magen-DarmTrakt.

14. Verbindung zur Verwendung des Anspruchs 13, wobei die Prostaglandin-PGE2-vermittelten Krankheiten ausgewählt sind aus der Gruppe, bestehend aus Autoimmunerkrankungen, Allergien, Entzündungen, Knochenerkrankungen, akuten oder chronischen Schmerzen und Tumoren.

15. Verbindung zur Verwendung des Anspruchs 14, wobei der Tumor ausgewählt ist aus der Gruppe bestehend aus Leberkrebs, Lungenkrebs, Prostatakrebs, Hautkrebs, Darmkrebs, Bauchspeicheldrüsenkrebs, Brustkrebs, Leukämie, Lymphom, Eierstockkrebs, Magenkrebs, Blasenkrebs, Nierenkrebs, Mundkrebs, Melanom, Speiseröhrenkrebs, Lymphom und Gebärmutterhalskrebs.

## Revendications

1. Composé de la formule (I) : 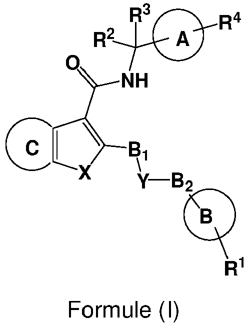 ou sels ou hydrates pharmaceutiquement acceptables de celle-ci, dans laquelle :
Ⓐ et Ⓑ sont chacun indépendamment choisis dans le groupe constitué par un cycle carboné en C3-C6, un cycle benzénique et un cycle hétéroaromatique à 5 ou 6 éléments doté d'un ou plusieurs atomes de O, N, S, dans laquelle Ⓐ et Ⓑ peuvent être éventuellement substitués par 1 à 3 substituants R⁵ ;
Ⓒ est un cycle substitué ou non substitué choisi dans le groupe constitué par un cycle hétérocyclique saturé de 4 à 7 éléments, dans laquelle le cycle hétérocyclique comprend un ou plusieurs hétéroatomes choisis parmi O ;
X représente -S- ;
Y est absent, ou un groupe choisi dans le groupe constitué par -CH₂-, -O-, -S-, -SO-, -SO₂-, et -N(R⁸)- ;
B₁ et B₂ sont des groupes choisis chacun indépendamment dans le groupe constitué par un absent, un alkylène en C1-C6, un alcénylène en C2-C6, un alcynylène en C2-C6 ; et B₁, B₂ et Y ne sont pas absents en même temps ;
R¹ est un ou plusieurs groupes choisis dans le groupe constitué par H, un alkyle en C1-C6, un halogène, un nitro, -N(R⁹)(R¹⁰), -OH, -CN, un haloalkyle en C₁-C₆, un alcoxy en C1-C6 et un haloalcoxy en C1-C6 ;
R² et R³ sont chacun indépendamment choisis dans le groupe constitué de H, d'un halogène, d'un alkyle en C1-C6, d'un haloalkyle en C1-C6 et d'un cycloalkyle en C3-C6 ; ou R², R³ forment avec l'atome de carbone auquel ils sont attachés un cycle de 3 à 6 éléments, qui est un cycle carboné ou un cycle hétérocyclique comprenant 1 à 3 hétéroatomes choisis parmi O, S ou N(R¹¹) ;
R⁴ représente un groupe COOR¹² ;
R⁵ est choisi parmi : H, halogène, alkyle en C1-C6, haloalkyle en C1-C6, cycloalkyle en C3-C6, et alcoxy en C1-C6 ;
R¹² est sélectionné dans le groupe constitué par un H et un alkyle en C1-C6 ;
R⁸, R⁹, R¹⁰ et R¹¹ sont sélectionnés indépendamment parmi : H, alkyle en C1-C6, haloalkyle en C1-C6, cycloalkyle en C3-C6, halocycloalkyle en C3-C6, aryle en C6-C10, hétéroaryle à 5 ou 6 éléments, 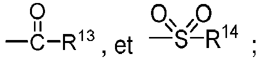
sauf indication contraire, un ou plusieurs atomes d'hydrogène des groupes substitués sont substitués par un ou plusieurs substituants choisis dans le groupe constitué par F, Cl, Br, I, hydroxyle, méthyle, éthyle, isopropyle, méthoxy, éthoxy, trifluorométhyle, difluorométhoxy, trifluorométhoxy, nitro, -CN, oxo (= O) ;
R¹³ et R¹⁴ sont chacun indépendamment sélectionnés parmi : H, alkyle en C1-C6, aryle en C6-C10, alkylène en C1-C6 et aryle en -C6-C10.

2. Composé selon la revendication 1, ou ses sels ou hydrates pharmaceutiquement acceptables, dans lequel Ⓒ est un oxa-hétérocycle à 6 éléments substitué ou non substitué.

3. Composé selon la revendication 1, ou ses sels ou hydrates pharmaceutiquement
acceptables, dans lequel 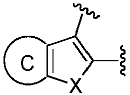 présente une structure telle que représentée dans la formule suivante : 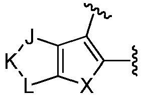
dans laquelle l'un de J, K et L est -O- ; et le reste de J, K et L sont choisis dans le groupe constitué par -CH₂-, -CH(CH₃)-, -CH(CH₂CH₃)-, -C(CH₃)₂-, 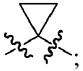
ou 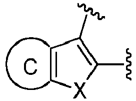 présente une structure telle que représentée dans la formule suivante : 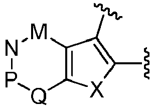
dans laquelle l'un de M, N, P et Q est -O- ; et le reste de M, N, P et Q sont chacun indépendamment choisis dans le groupe constitué par -CH₂-, -CH(CH₃)-, -CH(CH₂CH₃)-, -C(CH₃)2-, et 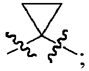
ou 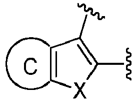 présente une structure telle que représentée dans la formule suivante : 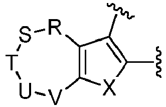
dans laquelle l'un de R, S, T, U et V est -O- ; et le reste de R, S, T, U et V sont chacun indépendamment choisis dans le groupe constitué par -CH2-, -CH(CH3)-, -CH(CH2CH3)-, -C(CH₃)2-, et

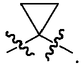

4. Composé selon la revendication 1, ou ses sels ou hydrates pharmaceutiquement acceptables, dans lequel 1 ou 2 atomes d'hydrogène sur le cycle Ⓒ sont substitués par un substituant choisi dans le groupe constitué par le méthyle, l'éthyle et l'isopropyle.

5. Composé selon la revendication 1, ou ses sels ou hydrates pharmaceutiquement
acceptables, dans lequel 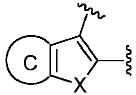 présente une structure telle que représentée dans la formule suivante : 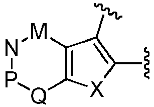
dans laquelle l'un des M, N, P et Q est -O- ; et le reste des M, N, P et Q sont chacun indépendamment choisis dans le groupe constitué par -CH2-, -CH(CH3)-, -CH(CH2CH3)-, -C(CH3)2-et 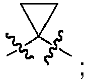 et
X représente -S-.

6. Composé selon la revendication 1, ou ses sels ou hydrates pharmaceutiquement acceptables, dans lequel Ⓐ et Ⓑ sont chacun indépendamment choisis dans le groupe constitué par un cycle benzénique et un cycle hétéroaromatique à 5 ou 6 éléments comprenant un ou plusieurs atomes de O, N, S, dans lequel Ⓐ et Ⓑ peuvent éventuellement être substitués par 1 à 3 substituants R⁵.

7. Composé selon la revendication 1, ou ses sels ou hydrates pharmaceutiquement acceptables, dans lequel Ⓐ et Ⓑ sont chacun indépendamment choisis dans le groupe constitué par un cycle benzénique et un cycle hétéroaromatique à 5 ou 6 éléments, dans lequel le cycle hétéroaromatique à 5 ou 6 éléments est choisi dans le groupe constitué par la pyridine, la pyrimidine, le thiazole, l'isothiazole, le furane, le thiophène et le pyrrole ; dans lequel Ⓐ et Ⓑ peuvent éventuellement être substitués par 1 à 3 substituants R⁵.

8. Composé selon la revendication 1, ou ses sels ou hydrates pharmaceutiquement acceptables, dans lequel R⁴ est choisi parmi l'un quelconque des groupes suivants : -COOH, -COOCH3, -COOCH2CH3, -COOCH2CH2CH3, -COOCH(CH₃)2.

9. Composé selon la revendication 1 ou ses sels ou hydrates pharmaceutiquement acceptables, dans lequel,
B1 et B2 sont chacun indépendamment choisis dans le groupe constitué par - (CH2)ₙ-, dans lequel n=0, 1, 2, 3 ou 4, -CH=CH-, -CH=CH-CH₂-, -CH₂-CH=CH-, -CH=CH-CH₂-CH₂-, -CH2-CH=CH-CH₂-, -CH₂-CH₂-CH=CH- ; -C≡C-, -C≡C-CH2-, -CH₂-C≡C-, -C≡C-CH2-CH2-, -CH2-C≡C-CH2-, -CH2-CH2-C≡C-, à condition que B₁ et B₂ ne soient pas - (CH2)n- en même temps, dans lequel n=0.

10. Composé selon la revendication 1, ou ses sels ou hydrates pharmaceutiquement acceptables, dans lequel B2 est -(CH2)n-, dans lequel n=0, et B1 est choisi dans le groupe constitué par -(CH2)ₙ- ; dans lequel n=1, 2, 3 ou 4, -CH=CH-, -CH=CH-CH₂-, -CH₂-CH=CH-, -CH=CH-CH₂-CH₂-, -CH₂-CH=CH-CH₂-, -CH₂-CH₂-CH=CH- ; -C≡C-, -C≡ ; et Y est absent.

11. Composé selon la revendication 1, ou ses sels ou hydrates pharmaceutiquement acceptables, dans lequel le composé est sélectionné dans le groupe constitué par :

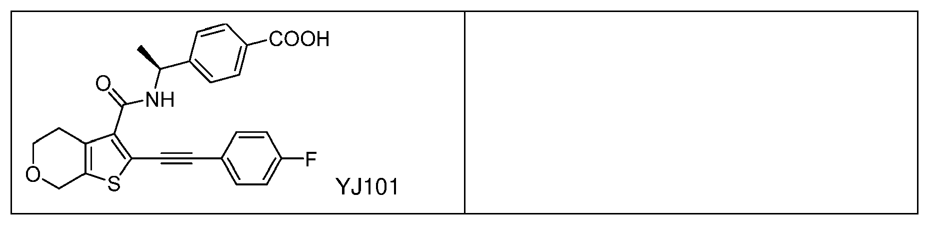

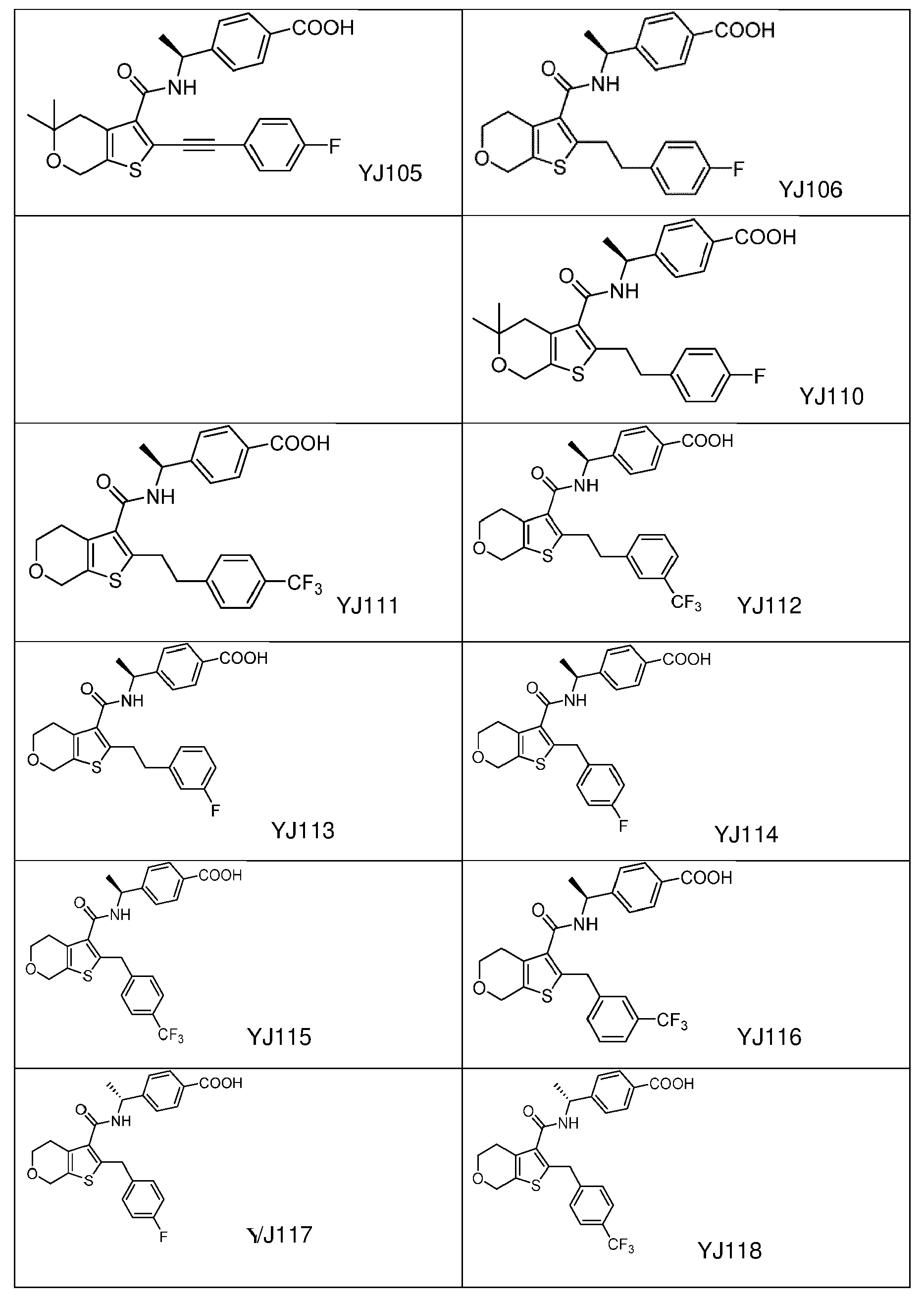

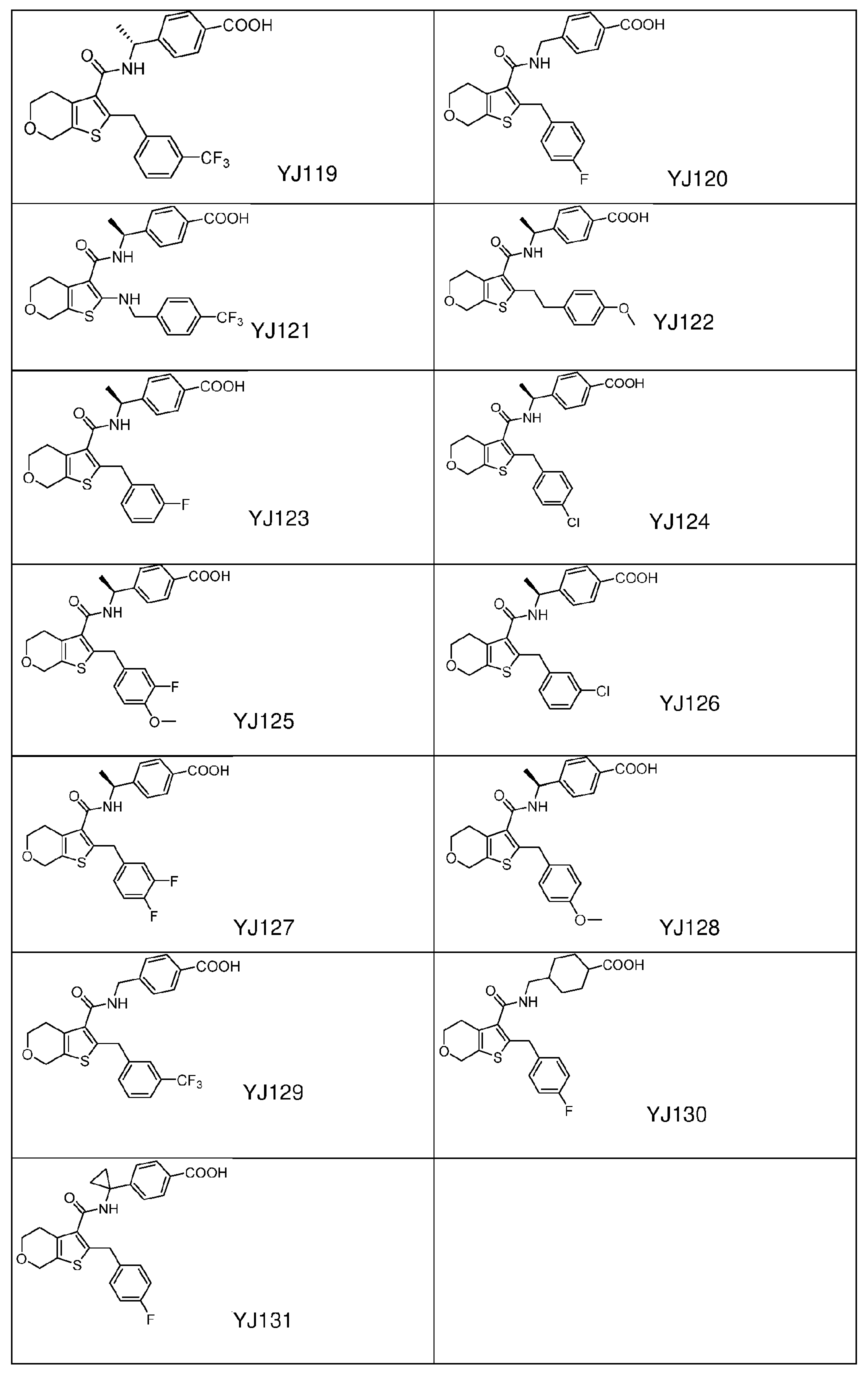

12. Composition pharmaceutique, dans laquelle la composition pharmaceutique comprend : une quantité thérapeutiquement efficace du composé de formule (I) selon la revendication 1, ou de ses sels ou hydrates pharmaceutiquement acceptables ; et des excipients pharmaceutiquement acceptables.

13. Composé de formule (I) selon la revendication 1, ou ses sels ou hydrates pharmaceutiquement acceptables pour une utilisation dans :
(i) l'antagonisme du récepteur de la prostaglandine -4 (EP4) ;
(ii) l'inhibition de la prostaglandine PGE 2 ;
(iii) l'amélioration de l'immunocompétence *in vivo* de l'homme ou des mammifères, ou la prévention et/ou le traitement des maladies médiées par la prostaglandine PGE2 ;
(iv) la réduction des effets secondaires des anti-inflammatoires non stéroïdiens et des inhibiteurs de la cyclo-oxygénase-2 sur le système cardiovasculaire et le système gastro-intestinal.

14. Composé pour l'utilisation de la revendication 13, dans lequel les maladies médiées par la prostaglandine PGE2 sont choisies dans le groupe constitué par les maladies autoimmunes, l'allergie, l'inflammation, les maladies osseuses, la douleur aiguë ou chronique, et les tumeurs.

15. Composé pour l'utilisation de la revendication 14, dans lequel la tumeur est choisie dans le groupe constitué par le cancer du foie, le cancer du poumon, le cancer de la prostate, le cancer de la peau, le cancer du côlon, le cancer du pancréas, le cancer du sein, la leucémie, le lymphome, le cancer de l'ovaire, le cancer de l'estomac, le cancer de la vessie, le cancer du rein, le cancer de la bouche, le mélanome, le cancer de l'oesophage, le lymphome et le cancer du col de l'utérus.
